# EUROPEAN PATENT APPLICATION

(11) **EP 1 686 189 A2**
(43) Date of publication of application: **02.08.2006**
(21) Application number: 05258108.9
(22) Date of filing: 29.12.2005
(51) Int. Cl.: C12Q 1/68, C07H 21/00

(54) **Detection of polynucleotides on nucleic acid arrays using azido-modified triphosphate nucleotide analogs**

(30) Priority: 30.12.2004 US 640387 P
(71) Applicant: Affymetrix, Inc., Santa Clara, CA 95051 (US)
(72) Inventor: Barone, Anthony D., San Jose, CA 95125 (US); Li, Handong, San Jose, CA 95129 (US); McGall, Glenn H., Palo Alto, CA 94303 (US)
(74) Representative: Bizley, Richard Edward

(57) **Abstract**

Methods are provided for detecting hybridization of a polynucleotide to a nucleic acid array by chemically modifying the polynucleotide to contain a detectable label. According to one aspect of the present invention, a method is provided for detecting the presence of a mRNA in a nucleic acid sample, the method having the steps of providing a mRNA sample and azido modified nucleotides, hybridizing a primer to the mRNA, reversed transcribing the mRNA to provide azido modified DNA, followed by reacting the azido groups with a detectable label, hybridizing the labeled RNA to a nucleic acid array and detecting the presence of the mRNA.

Still other methods are provided for detecting the presence or absence of a polynucleotide of interest on a nucleic acid array, the method having the steps of providing a nucleic acid sample comprising a polynucleotide; providing an enzyme to amplify the polynucleotide using an azido nucleotide derivative; amplifying said polynucleotide to provide azido labeled amplified nucleic acids; reacting the azido groups on said nucleic acids with a detectable label to provide labeled nucleic acids; hybridizing said amplified nucleic acids to a nucleic acid array; and detecting the presence or absence of said polynucleotide.

Still other methods are presented for detecting polynucleotides on a nucleic acid array using ligases and terminal transferases to end label polynucleotides.

## Description

### FIELD OF THE INVENTION

The present invention relates generally to the field of nucleic acid arrays. More specifically, the present invention relates to chemical reactions for joining different moieties to detectable labels. The present invention relates to compounds and methods for the chemical modification and detection of nucleic acids on a nucleic acid array.

### BACKGROUND OF THE INVENTION

Gene expression in diseased and healthy individuals is oftentimes different and characterizable. The ability to monitor gene expression in such cases provides medical professionals with a powerful diagnostic tool. This form of diagnosis is especially important in the area of oncology, where it is thought that the overexpression of an oncogene, or the under expression of a tumor suppressor gene, results in tumor genesis. See Mikkelson et al. *J. Cell. Biochem.* **1991,** *46,* 3-8.

One can indirectly monitor gene expression, for example, by measuring nucleic acid (e.g., mRNA) that is the transcription product of a targeted gene. The nucleic acid is chemically or biochemically labeled with a detectable moiety and allowed to hybridize with a localized nucleic acid of known sequence sometimes, know here as a probe. The detection of a labeled nucleic acid at the probe position indicates that the targeted gene has been expressed. See International Application Publication Nos.WO 97/27317, WO 92/10588 and WO 97/10365.

### SUMMARY OF THE INVENTION

Methods are presented for the detection of polynucleotides on a nucleic acid array wherein the polynucleotides are chemically modified to contain a detectable label. According to one aspect of the present invention, methods are presented for detecting the presence of a mRNA in a nucleic acid sample on a nucleic acid array using post-amplification chemical labeling, the method having the steps of providing a nucleic acid sample comprising mRNA; hybridizing the mRNA with an oligonucleotide; providing a nucleotide derivative having a reactive orthogonal group allowing for the chemical attachment of a detectable label; reverse transcribing the mRNA with a reverse transcriptase and the derivative to provide DNA homologous to all or part of said mRNA; reacting the orthogonal groups on the cRNA with a detectable label to provide labeled cRNA; and hybridizing the labeled cRNA to the nucleic acid array to detect the presence or absence of the mRNA. According to another aspect of the present invention, the RNA may optionally be amplified by using an oligonucleotide probe having a T7 RNA polymerase promoter. The T7 RNA polymerase promoter can be used to provide labeled cRNA.

In another aspect of the present invention, methods are presented for detecting the presence or absence of a polynucleotide of interest on a nucleic acid array, the method having the steps of providing a nucleic acid sample comprising a polynucleotide; providing an enzyme to amplify the polynucleotide with a nucleotide derivative having a reactive orthogonal group; amplifying the polynucleotide to provide amplified nucleic acids having reactive orthogonal groups (e.g. azido groups); reacting said orthogonal groups on said nucleic acids with a detectable label to provide labeled nucleic acids; hybridizing said amplified nucleic acids to a nucleic acid array; and detecting the presence or absence of said polynucleotide.

### DETAILED DESCRIPTION OF THE INVENTION

As used in this application, the singular form "a," "an," and "the" include plural references unless the context clearly dictates otherwise. For example, the term "an agent" includes a plurality of agents, including mixtures thereof.

An individual is not limited to a human being but may also be other organisms including but not limited to mammals, plants, bacteria, or cells derived from any of the above.

Throughout this disclosure, various aspects of this invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

The practice of the present invention may employ, unless otherwise indicated, conventional techniques and descriptions of organic chemistry, polymer technology, molecular biology (including recombinant techniques), cell biology, biochemistry, and immunology, which are within the skill of the art. Such conventional techniques include polymer array synthesis, hybridization, ligation, and detection of hybridization using a label. Specific illustrations of suitable techniques can be had by reference to the example herein below. However, other equivalent conventional procedures can, of course, also be used. Such conventional techniques and descriptions can be found in standard laboratory manuals such as *Genome Analysis: A Laboratory Manual Series (Vols. I-IV), Using Antibodies: A Laboratory Manual, Cells: A Laboratory Manual, PCR Primer: A Laboratory Manual,* and Molecular *Cloning: A Laboratory Manual* (all from Cold Spring Harbor Laboratory Press), Stryer, L. (1995) *Biochemistry* (4th Ed.) Freeman, New York, *Gait, "Oligonucleotide Synthesis: A Practical Approach" 1984, IRL Press, London,* Nelson and Cox (2000), *Lehninger, Principles of Biochemistry* 3^{rd} Ed., W.H. Freeman Pub., New York, NY and Berg et al. (2002) *Biochemistry,* 5^{th} Ed., W.H. Freeman, Pub., New York, NY, all of which are herein incorporated in their entirety by reference for all purposes.

The present invention can employ solid substrates, including arrays in some preferred embodiments. Methods and techniques applicable to polymer (including protein) array synthesis have been described in U.S.S.N 09/536,841, WO 00/58516, U.S. Patents Nos. 5,143,854, 5,242,974, 5,252,743, 5,324,633, 5,384,261, 5,405,783, 5,424,186, 5,451,683, 5,482,867, 5,491,074, 5,527,681, 5,550,215, 5,571,639, 5,578,832, 5,593,839, 5,599,695, 5,624,711, 5,631,734, 5,795,716, 5,831,070, 5,837,832, 5,856,101, 5,858,659, 5,936,324, 5,968,740, 5,974,164, 5,981,185, 5,981,956, 6,025,601, 6,033,860, 6,040,193, 6,090,555, 6,136,269, 6,269,846 and 6,428,752, in PCT Applications Nos. PCT/US99/00730 (International Publication Number WO 99/36760) and PCT/US01/04285, which are all incorporated herein by reference in their entirety for all purposes.

Patents that describe synthesis techniques in specific embodiments include U.S. Patents Nos. 5,412,087, 6,147,205, 6,262,216, 6,310,189, 5,889,165, and 5,959,098. Nucleic acid arrays are described in many of the above patents, but the same techniques are applied to polypeptide arrays.

The present invention also contemplates many uses for polymers attached to solid substrates. These uses include gene expression monitoring, profiling, library screening, genotyping and diagnostics. Gene expression monitoring, and profiling methods can be shown in U.S. Patents Nos. 5,800,992, 6,013,449, 6,020,135, 6,033,860, 6,040,138, 6,177,248 and 6,309,822. Genotyping and uses therefore are shown in USSN 60/319,253, 10/013,598, and U.S. Patents Nos. 5,856,092, 6,300,063, 5,858,659, 6,284,460, 6,361,947, 6,368,799 and 6,333,179. Other uses are embodied in U.S. Patents Nos. 5,871,928, 5,902,723, 6,045,996, 5,541,061, and 6,197,506.

The present invention also contemplates sample preparation methods in certain preferred embodiments. Prior to or concurrent with genotyping, the genomic sample may be amplified by a variety of mechanisms, some of which may employ PCR*. See,* e.g., *PCR Technology: Principles and Applications for DNA Amplification* (Ed. H.A. Erlich, Freeman Press, NY, NY, 1992); *PCR Protocols: A Guide to Methods and Applications* (Eds. Innis, et al., Academic Press, San Diego, CA, 1990); Mattila et al., *Nucleic Acids Res.* 19, 4967 (1991); Eckert et al., *PCR Methods and Appl ications 1,* 17 (1991); *PCR* (Eds. McPherson et al., IRL Press, Oxford); and U.S. Patent Nos. 4,683,202, 4,683,195, 4,800,159 4,965,188,and 5,333,675, and each of which is incorporated herein by reference in their entireties for all purposes. The sample may be amplified on the array. See, for example, U.S Patent No 6,300,070 and U.S. patent application 09/513,300, which are incorporated herein by reference.

Other suitable amplification methods include the ligase chain reaction (LCR) *(e.g.,* Wu and Wallace, *Genomics* 4, 560 (1989), Landegren et al., *Science* 241, 1077 (1988) and Barringer et al. *Gene* 89:117 (1990)), transcription amplification (Kwoh et al., *Proc. Natl. Acad. Sci. USA* 86, 1173 (1989) and WO88/10315), self-sustained sequence replication (Guatelli et al., *Proc. Nat. Acad. Sci. USA,* 87, 1874 (1990) and WO90/06995), selective amplification of target polynucleotide sequences (U.S. Patent No 6,410,276), consensus sequence primed polymerase chain reaction (CP-PCR) (U.S. Patent No 4,437,975), arbitrarily primed polymerase chain reaction (AP-PCR) (U.S. Patent No 5, 413,909, 5,861,245) and nucleic acid based sequence amplification (NABSA). *(See,* US patents nos. 5,409,818, 5,554,517, and 6,063,603, each of which is incorporated herein by reference). Other amplification methods that may be used are described in, U.S. Patent Nos. 5,242,794, 5,494,810, 4,988,617 and in USSN 09/854,317, each of which is incorporated herein by reference.

Additional methods of sample preparation and techniques for reducing the complexity of a nucleic sample are described in Dong et al., *Genome Research* 11, 1418 (2001), in U.S. Patent No 6,361,947, 6,391,592 and U.S. Patent application Nos. 09/916,135, 09/920,491, 09/910,292, and 10/013,598.

Methods for conducting polynucleotide hybridization assays have been well developed in the art. Hybridization assay procedures and conditions will vary depending on the application and are selected in accordance with the general binding methods known including those referred to in: Maniatis et al. *Molecular Cloning: A Laboratory Manual* (2^{nd} Ed. Cold Spring Harbor, N.Y, 1989); Berger and Kimmel *Methods in Enzymology,* Vol. 152, *Guide to Molecular Cloning Techniques* (Academic Press, Inc., San Diego, CA, 1987); Young and Davism, *P.N.A.S,* 80: 1194 (1983). Methods and apparatus for carrying out repeated and controlled hybridization reactions have been described in US patent 5,871,928, 5,874,219, 6,045,996 and 6,386,749, 6,391,623 each of which are incorporated herein by reference

The present invention also contemplates signal detection of hybridization between ligands in certain preferred embodiments. See U.S. Pat. Nos. 5,143,854, 5,578,832; 5,631,734; 5,834,758; 5,936,324; 5,981,956; 6,025,601; 6,141,096; 6,185,030; 6,201,639; 6,218,803; and 6,225,625, in U.S. Patent application 60/364,731 and in PCT Application PCT/US99/06097 (published as WO99/47964), each of which also is hereby incorporated by reference in its entirety for all purposes.

Methods and apparatus for signal detection and processing of intensity data are disclosed in, for example, U.S. Patents Numbers 5,143,854, 5,547,839, 5,578,832, 5,631,734, 5,800,992, 5,834,758; 5,856,092, 5,902,723, 5,936,324, 5,981,956, 6,025,601, 6,090,555, 6,141,096, 6,185,030, 6,201,639; 6,218,803; and 6,225,625, in U.S. Patent application 60/364,731 and in PCT Application PCT/US99/06097 (published as WO99/47964), each of which also is hereby incorporated by reference in its entirety for all purposes.

The practice of the present invention may also employ conventional biology methods, software and systems. Computer software products of the invention typically include computer readable medium having computer-executable instructions for performing the logic steps of the method of the invention. Suitable computer readable medium include floppy disk, CD-ROM/DVD/DVD-ROM, hard-disk drive, flash memory, ROM/RAM, magnetic tapes and etc. The computer executable instructions may be written in a suitable computer language or combination of several languages. Basic computational biology methods are described in, e.g. Setubal and Meidanis et al., *Introduction to Computational Biology Methods* (PWS Publishing Company, Boston, 1997); Salzberg, Searles, Kasif, (Ed.), *Computational Methods in Molecular Biology,* (Elsevier, Amsterdam, 1998); Rashidi and Buehler, *Bioinformatics Basics: Application in Biological Science and Medicine* (CRC Press, London, 2000) and Ouelette and Bzevanis *Bioinformatics: A Practical Guide for Analysis of Gene and Proteins* (Wiley & Sons, Inc., 2^{nd} ed., 2001).

The present invention may also make use of various computer program products and software for a variety of purposes, such as probe design, management of data, analysis, and instrument operation. See, U.S. Patent Nos. 5,593,839, 5,795,716, 5,733,729, 5,974,164, 6,066,454, 6,090,555, 6,185,561, 6,188,783, 6,223,127, 6,229,911 and 6,308,170.

Additionally, the present invention may have preferred embodiments that include methods for providing genetic information over networks such as the Internet as shown in U.S. Patent applications 10/063,559, 60/349,546, 60/376,003, 60/394,574, 60/403,381.

One of skill in the art will appreciate that in order to measure the transcription level (and thereby the expression level) of a gene or genes, it is desirable to provide a nucleic acid sample comprising mRNA transcript(s) of the gene or genes, or nucleic acids derived from the mRNA transcript(s). As used herein, a nucleic acid derived from a mRNA transcript refers to a nucleic acid which is homologous to the mRNA or to an anti-sense strand homologous to the mRNA .

Thus, a cDNA reverse transcribed from a mRNA, an RNA transcribed from that cDNA, a DNA amplified from the cDNA, an RNA transcribed from the amplified DNA, etc., are all derived from the mRNA transcript and detection of such derived products is indicative of the presence and/or abundance of the original transcript in a sample. Thus, suitable samples include, but are not limited to, mRNA transcripts, cDNA reverse transcribed from the mRNA, cRNA transcribed from the cDNA, DNA reverse transcribed from cRNA and the like.

In a particularly preferred embodiment, where it is desired to quantify the transcription level (and thereby expression) of a one or more genes in a sample, the nucleic acid sample is one in which the concentration of the mRNA transcript(s) of the gene or genes, or the concentration of the nucleic acids derived from the mRNA transcript(s), is proportional to the transcription level (and therefore expression level) of that gene. Similarly, it is preferred that the hybridization signal intensity be proportional to the amount of hybridized nucleic acid. While it is preferred that the proportionality be relatively strict (e.g., a doubling in transcription rate results in a doubling in mRNA transcript in the sample nucleic acid pool and a doubling in hybridization signal), one of skill will appreciate that the proportionality can be more relaxed and even non-linear. Thus, for example, an assay where a 5 fold difference in concentration of the target mRNA results in a 3 to 6 fold difference in hybridization intensity is sufficient for most purposes. Where more precise quantification is required appropriate controls can be run to correct for variations introduced in sample preparation and hybridization as described herein. In addition, serial dilutions of "standard" target mRNAs can be used to prepare calibration curves according to methods well known to those of skill in the art. Of course, where simple detection of the presence or absence of a transcript is desired, no elaborate control or calibration is required.

In the simplest embodiment, such a nucleic acid sample is the total mRNA isolated from a biological sample. However, this does not need to be the case. For example, individual subpopulations of mRNA or combinations thereof are also envisaged. The term "biological sample", as used herein, refers to a sample obtained from an organism or from components (e.g., cells) of an organism. The sample may be of any biological tissue or fluid. Frequently the sample will be a "clinical sample" which is a sample derived from a patient. Such samples include, but are not limited to, sputum, blood, blood cells (e.g., white cells), tissue or fine needle biopsy samples, urine, peritoneal fluid, and pleural fluid, or cells there from. Biological samples may also include sections of tissues such as frozen sections taken for histological purposes.

The nucleic acid (either genomic DNA or mRNA) may be isolated from the sample according to any of a number of methods well known to those of skill in the art. One of skill will appreciate that where alterations in the copy number of a gene are to be detected genomic DNA is preferably isolated. Conversely, where expression levels of a gene or genes are to be detected, preferably RNA (mRNA) is isolated.

Methods of isolating total mRNA are well known to those of skill in the art. For example, methods of isolation and purification of nucleic acids are described in detail in Chapter 3 of Laboratory Techniques in Biochemistry and Molecular Biology: Hybridization With Nucleic Acid Probes, Part I. Theory and Nucleic Acid Preparation, P. Tijssen, ed. Elsevier, N.Y. (1993) and Chapter 3 of Laboratory Techniques in Biochemistry and Molecular Biology: Hybridization with Nucleic Acid Probes, Part I. Theory and Nucleic Acid Preparation, P. Tijssen, ed. Elsevier, N.Y. (1993)).

According to an aspect of the present invention, total nucleic acid is isolated from a given sample using, for example, an acid guanidinium-phenol-chloroform extraction method and polyA⁺ mRNA is isolated by oligo dT column chromatography or by using (dT)n magnetic beads (see, e.g., Sambrook et al., Molecular Cloning: A Laboratory Manual (2nd ed.), Vols. 1-3, Cold Spring Harbor Laboratory, (1989), or Current Protocols in Molecular Biology, F. Ausubel et al., ed. Greene Publishing and Wiley-Interscience, New York (1987)).

Frequently, it is desirable to amplify the nucleic acid sample prior to hybridization. One of skill in the art will appreciate that whatever amplification method is used, if a quantitative result is desired, care must be taken to use a method that maintains or controls for the relative frequencies of the amplified nucleic acids.

Methods of "quantitative" amplification are well known to those of skill in the art. For example, quantitative PCR involves simultaneously co-amplifying a known quantity of a control sequence using the same primers. This provides an internal standard that may be used to calibrate the PCR reaction. The high density array may then include probes specific to the internal standard for quantification of the amplified nucleic acid.

One preferred internal standard is a synthetic AW 106 cRNA. The AW 106 cRNA is combined with RNA isolated from the sample according to standard techniques known to those of skill in the art. The RNA is then reverse transcribed using a reverse transcriptase to provide copy DNA. The cDNA sequences are then amplified (e.g., by PCR) using labeled primers. The amplification products are separated, typically by electrophoresis, and the amount of radioactivity (proportional to the amount of amplified product) is determined. The amount of mRNA in the sample is then calculated by comparison with the signal produced by the known AW 106 RNA standard. Detailed protocols for quantitative PCR are provided in PCR Protocols, A Guide to Methods and Applications, Innis et al., Academic Press, Inc. N.Y., (1990).

Other suitable amplification methods include, but are not limited to polymerase chain reaction (PCR) (Innis, et al., PCR Protocols. A guide to Methods and Application. Academic Press, Inc. San Diego, (1990)), ligase chain reaction (LCR) (see Wu and Wallace, Genomics, 4: 560 (1989), Landegren, et al., Science, 241: 1077 (1988) and Barringer, et al., Gene, 89: 117 (1990), transcription amplification (Kwoh, et al., Proc. Natl. Acad. Sci. USA, 86: 1173 (1989)), and self-sustained sequence replication (Guatelli, et al., Proc. Nat. Acad. Sci. USA, 87: 1874 (1990)).

Methods of in vitro polymerization are well known to those of skill in the art (see, e.g., Sambrook, supra.) and this particular method is described in detail by Van Gelder, et al., Proc. Natl. Acad. Sci. USA, 87: 1663-1667 (1990) who demonstrate that in vitro amplification according to this method preserves the relative frequencies of the various RNA transcripts. Moreover, Eberwine et al. Proc. Natl. Acad. Sci. USA, 89: 3010-3014 provide a protocol that uses two rounds of amplification via in vitro transcription to achieve greater than 10⁶ fold amplification of the original starting material thereby permitting expression monitoring even where biological samples are limited.

It will be appreciated by one of skill in the art that the direct transcription method described above provides an antisense (aRNA) pool. Where antisense RNA is used as the target nucleic acid, the oligonucleotide probes provided in the array are chosen to be complementary to subsequences of the antisense nucleic acids. Conversely, where the target nucleic acid pool is a pool of sense nucleic acids, the oligonucleotide probes are selected to be complementary to subsequences of the sense nucleic acids. Finally, where the nucleic acid pool is double stranded, the probes may be of either sense as the target nucleic acids include both sense and antisense strands.

The protocols cited above include methods of generating pools of either sense or antisense nucleic acids. Indeed, one approach can be used to generate either sense or antisense nucleic acids as desired. For example, cDNA can be directionally cloned into a vector (e.g., Stratagene's p Bluscript II KS (+) phagemid) such that it is flanked by the T3 and T7 promoters. In vitro transcription with the T3 polymerase will produce RNA of one sense (the sense depending on the orientation of the insert), while in vitro transcription with the T7 polymerase will produce RNA having the opposite sense. Other suitable cloning systems include phage lamda vectors designed for Cre-loxP plasmid subcloning (see e.g., Palazzolo et al., Gene, 88: 25-36 (1990)).

In a particularly preferred embodiment, a high activity RNA polymerase (e.g. about 2500 units/.mu.L for T7, available from Epicentre Technologies) is used. Nucleic acid labeling

According to one aspect of the present invention, nucleic acids or polynucleotides are detected by the use of orthogonal reactive groups. An orthogonal reactive group is a chemical moiety which is not naturally present on the nucleic acid strand and which allows specific chemical reaction with molecule bearing a detectable moiety. In accordance with an aspect of the present invention, an azido group (-N=N=N) is an orthogonal group. Nucleic acids do not naturally contain such groups. In addition, azido groups can be reacted specifically with, for example, phosphanes and "Click" groups to allow specific reactions with a detectable moiety such as an appropriately modified biotin group. After biotin labeling, the labeled nucleic acid can be hybridized to a nucleic acid array to determine the presence or absence of a particular nucleic acid such as a mRNA or genomic DNA.

In accordance with an aspect of the present invention, the nucleic acid array may be fabricated in any number of ways. In a particularly preferred embodiment of the present invention, the GeneChip® Array of Affymetrix, Inc. can be used. One product offered by Affymetrix is an array of oligonucleotides fabricated on a solid surface using the techniques of photolithography. However, alternative array products are also preferred in accordance with the present invention. For example, spotted arrays of cDNAs or oligonucleotides are a preferred aspect of the present invention. Moreover, there is no requirement of a flat plate, oligo or polynucleotides for hybridization may be disposed upon beads in accordance with an aspect of the present invention

In considering a modified nucleotide as a substrate for the polymerases discussed above, those of skill in the art will recognize that modified nucleotides such as azido modified nucleotides may not be incorporated as efficiently as wild type nucleotides. There are several possible reasons for an azido modified nucleotide to react less efficiently than its non-azido counterpart. Considering the interaction of an enzyme, such as a polymerase, with a substrate, for example a nucleotide, it is possible that the placement of an azido group on a particular nucleotide might reduce the affinity of the enzyme for the substrate. For example, the enzyme might have a pocket for the nucleotide in which the nucleotide is held by various non-covalent forces, e.g., ionic bonding, hydrogen bonding, van der waals forces, etc. The azide moiety is ionic in nature: 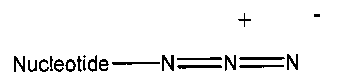 It is possible that introduction of this charged species could disrupt the enzyme's affinity for the nucleotide by affecting ionic bonding in the pocket. It is also possible that placement of the azido group in certain positions of the nucleotide might cause steric hindrance. However, persons of skill are familiar with a number of techniques which can readily solve such problems.

At least three targets present themselves for resolving issues concerning an azido substrate not working efficiently with an enzyme: modifying the substrate, modifying the enzyme and modifying the conditions under which the enzyme works. First, the azido nucleotide itself could be modified. For example, the azido group could be moved from one part of the molecule to another. If the 3 dimensional structure of the enzyme is known, one might be able to "rationally" modify the azido substrate, i.e., designing it in view of the three dimensional structure of the amino acids making up the protein and particularly those making up the pocket.. Alternatively, if the azido group is situated such that it blocks base pairing, it can be moved to another part of the molecule, for example to the sugar portion of the nucleotide or to part of the base that is not involved in hydrogen bonding with its counterpart in the opposing chain.

Another method of solving activity issues, generally the first one attempted if some activity is detected, is too modify the conditions the enzyme is used under. For example, if the azide-substrate is not incorporated well by the polymerase, one could simply increase the amount in the reaction. There are limits to this approach, however, Sometimes modified nucleotides can act as enzyme inhibitors. Other changes that can be easily made to the assay conditions are temperature, time, pH, amount of enzyme, ionic conditions, etc.

Yet another tool available to those of skill in the art is to modify the amino acids encoding an enzyme of interest. For example, DNA polymerase enzymes do not normally accept ribonuleotides. However, Gao et al., "Conferring RNA polymerase Activity to a DNA polymerase: A single residue in reverse transcriptase controls substrate selection" *Proc. Natl. Acad. Sci.* USA Vol. 94, pp. 407-411, January 1977) showed that changing a single amino acid would allow for reverse transcriptase to incorporate ribonucleotides. For example, Sande et al. showed that changing cofactors such as metal ions can cause an enzyme to accept a different substrate than it might. See Sande, et al., *J. Biol. Chem.* Vol. 247, No. 19 (1972).

In summary, there are numerous ways of dealing with a substrate which is not incorporated well by an enzyme of interest. These are well known techniques to those of skill in the art as shown above

In accordance with an aspect of the present invention aza modified nucleotides are disclosed as one form of modified nucleotide which can be specifically reacted with detectable labels.
Azido functional groups offer a number of advantages for chemical labeling of nucleic acids: 1) the azide moiety is absent in naturally occurring nucleic acids, i.e. it is "bioorthogonal;" 2) azido groups are highly reactive, but despite their high intrinsic reactivity, azides undergo a selective ligation with a very limited number of reactions partners; 3) the azide group is relatively small and can be introduced into a nucleotide without substantially altering the molecular size of the nucleotide. See, "The Staudinger Ligation - A Gift to Chemical Biology," Köhn, M. and Breinbauer, R., *Angew. Chem. Int. Ed.* 2004, 43, 3106 -3116; G. T. Hermanson, Bioconjugate Techniques, Academic Press,San Diego, 1996; H. C. Hang, C. R. Bertozzi, Acc. Chem. Res. 2001, 34, 727 - 736; W. G. Lewis, L. G. Green, F. Grynszpan, Z. Radic, P. R. Carlier, P. Taylor, M. G. Finn, K. B. Sharpless, Angew. Chem. 2002, 114, 1095 - 1099; Angew. Chem. Int. Ed. 2002, 41, 1053 -1057; V. V. Rostovtsev, L. G. Freen, V. V. Fokin, K. B. Sharpless, Angew. Chem. 2002, 114, 2708 - 2711; Angew. Chem. Int. Ed. 2002, 41, 2596 - 2599; C.W. Tornoe, C. Christensen, M. Meldal, J. Org. Chem. 2002, 67, 3057 - 3064; d) F. Fazio, M. C. Bryan, O. Blixt, J. C. Paulson, C.-H. Wong, J. Am. Chem. Soc. 2002, 124, 14 397 - 14402; e) Q. Wang, T.R. Chan, R. Hilgraf, V. V. Fokin, K. B. Sharpless, M. G. Finn, J. Am. Chem. Soc. 2003, 125, 3192 - 3193; R. Breinbauer, M. KLhn, ChemBioChem 2003, 4, 1147 - 1149; E. Saxon, C. R. Bertozzi, Science 2000, 287, 2007 - 2010; H. Staudinger, J. Meyer, Helv. Chim. Acta 1919, 2, 635 - 646; Y. G. Gololobov, I. N. Zhmurova, L. F. Kasukhin, Tetrahedron 1981, 37, 437 - 472; Y. G. Gololobov, L. F. Kasukhin, Tetrahedron 1992, 48, 1353 - 1406; P. M. Fresnada, P. Molina, Synlett 2004, 1 - 17; H. Staudinger, E. Hauser, Helv. Chim. Acta 1921, 4, 861; S. Luchansky, H. C. Hang, E. Saxon, J. R. Grunwell, C. Yu, D. H. Dube, C. R. Bertozzi, Methods Enzymol. 2003, 362, 249 - 272; E. Saxon, S. Luchansky, H. C. Hang, C. Yu, S. C. Lee, C. R. Bertozzi, J. Am. Chem. Soc. 2002, 124, 14893 - 14 902; E. Saxon, J. I. Armstrong, C. R. Bertozzi, Org. Lett. 2000, 2, 2141 - 2143; B. L. Nilsson, L. L. Kiessling, R. T. Raines, Org. Lett. 2000, 2, 1939 - 1941; B. L. Nilsson, L. L. Kiessling, R. T. Raines, Org. Lett. 2001, 3, 9 - 12; M. B. Soellner, B. L. Nilsson, R. T. Raines, J. Org. Chem. 2002, 67, 4993 - 4996; G. J. Cotton, T.W. Muir, Chem. Biol. 1999, 6, R247-R256; T. Wieland, E. Bokelmann, L. Bauer, H. U. Lang, H. Lau,Justus Liebigs Ann. Chem. 1953, 583, 129 - 149; P. E. Dawson,Scheme 16. Preparation of a library of azide-terminated small molecules and their immobilization on phosphane-decorated glass slides for thepreparation of small-molecule arrays. SPOS=solid-phase organic synthesis.Bioorganic Chemistry Angewandte Chemie, 3115 Angew. Chem. Int. Ed. 2004, 43, 3106-3116 www.angewandte.org _ 2004 Wiley-VCH Verlag GmbH & Co. KGaA; WeinheimT.W. Muir, 1. Clark-Lewis, S. B. H. Kent, Science 1994, 266, 776 - 779; J. Zaloom, D. C. Roberts, J. Org. Chem. 1981, 46, 5173 - 5176; J. T. Lundquist IV, J. C. Pelletier, Org. Lett. 2001, 3, 781 - 783; P. T. Nyffeler, C.-H. Liang, K. M. Koeller, C.-H.Wong, J. Am.Chem. Soc. 2002, 124, 10773 - 10 778; E. F. V. Scriven, K. Turnbull, Chem. Rev. 1988, 88, 297 - 368; B. L. Nilsson, R. J. Hondal, M. B. Soellner, R. T. Raines, J. Am.Chem. Soc. 2003, 125, 5268 - 5269; Merkx, D. T. S. Rijkers, J. Kemmink, R. M. J. Liskamp, Tetrahedron Lett. 2003, 44, 4515 - 4518; O. David, W. J. N. Meester, H. BierNugel, H. E. Schoemaker, H. Hiemstra, J. H. van Maarseveen, Angew. Chem. 2003, 115, 4509 - 4511; Angew. Chem. Int. Ed. 2003, 42, 4373-4375; K. L. Kiick, E. Saxon, D. A. Tirrell, C. R. Bertozzi, Proc. Natl. Acad. Sci. USA 2002, 99, 19 - 2; A. Lemieux, C. L. de Graffenried, C. R. Bertozzi, J. Am. Chem. Soc. 2003, 125, 4708 - 4709.C. C.-Y. Wang, T. S. Seo, Z. Li, H. Ruparel, J. Ju, Bioconjugate Chem. 2003, 14, 697 - 701; A. E. Speers, B. F. Cravatt, ChemBioChem 2004, 5, 41 - 47; H. Ovaa, P. F. van Swieten, B. M. Kessler, M. A. Leeuwenburgh, E. Fiebiger, A. M. C. H. van den Nieuwendijk, P. J. Galardy, G. A. van der Marel, H. L. Ploegh, H. S. Overkleeft, Angew.Chem. 2003, 115, 3754 - 3757; Angew. Chem. Int. Ed. 2003, 42, 3626 - 3629; H. C. Hang, C. Yu, D. L. Kato, C. R. Bertozzi, Proc. Natl. Acad. Sci. USA 2003, 100, 14846 - 14851; H. C. Hang, C. Yu, M. R. Pratt, C. R. Bertozzi, J. Am. Chem. Soc. 2004, 126, 6-7; M. B. Soellner, K. A. Dickson, B. L. Nilsson, R. T. Raines, J. Am. Chem. Soc. 2003, 125, 11790 - 11 791; and M. KLhn, R. Wacker, C. Peters, H. SchrLder, L. Soulere, R. Breinbauer, C. M. Niemeyer, H. Waldmann, Angew. Chem. 2003, 115, 6010 - 6014; Angew. Chem. Int. Ed. 2003, 42, 5830 -5834.

The reaction of azides with triaryl phosphanes to form iminophosphoranes was first reported in 1919 (see Scheme I, *infra).* H. Staudinger, J. Meyer, Helv. Chim. Acta 1919, 2, 635 - 646.

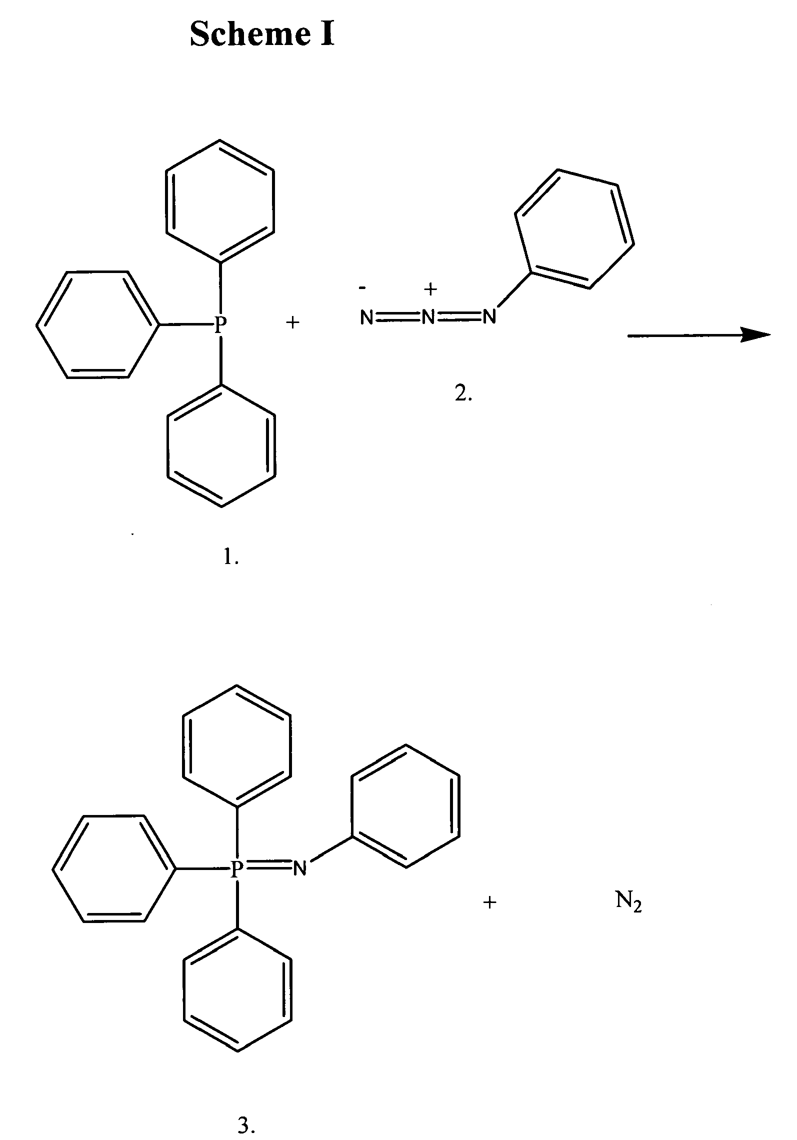

The product of the reaction of an azide with a phosphane, the aza-ylide 3, undergoes spontaneous hydrolysis to the amine and phosphane oxide in an aqueous environment. Saxon and Bertozzi postulated that a ligand would capture the nucleophilic aza-ylide 14 by intramolecular cyclization (see Scheme 4 of Köhn et al., *supra)* based on the rationale that an appropriately located electrophilic trap, such as an ester moiety, within the structure of the phosphane, would ultimately produce a stable amide bond before the competing aza-ylide hydrolysis could take place. Mechanistic studies by ³¹P NMR spectroscopy identified the aza-ylide 14 and the oxaphosphetane 15 as intermediates in the ligation reaction. E. Saxon, S. Luchansky, H. C. Hang, C. Yu, S. C. Lee, C. R. Bertozzi, J. Am. Chem. Soc. 2002, 124, 14893 - 14 902.

Still another modification of the basic reaction in which an amide bond is formed between the two coupling partners to give a product without a triaryl phosphane oxide moiety has been reported and, in the context of the instant invention, appears more promising than that reported immediately above. Bertozzi and co-workers (E. Saxon, J. I. Armstrong, C. R. Bertozzi, Org. Lett. 2000, 2, 2141 - 2143) and―in a parallel effort― Raines and coworkers (a. B. L. Nilsson, L. L. Kiessling, R. T. Raines, Org. Lett. 2000, 2, 1939 - 1941; b. B. L. Nilsson, L. L. Kiessling, R. T. Raines, Org. Lett. 2001, 3, 9 - 12; c. M. B. Soellner, B. L. Nilsson, R. T. Raines, J. Org. Chem. 2002, 67, 4993 - 4996) reported what is termed a "traceless" Staudinger ligation, in which the phosphane oxide moiety is cleaved during the hydrolysis (see Scheme II, infra):

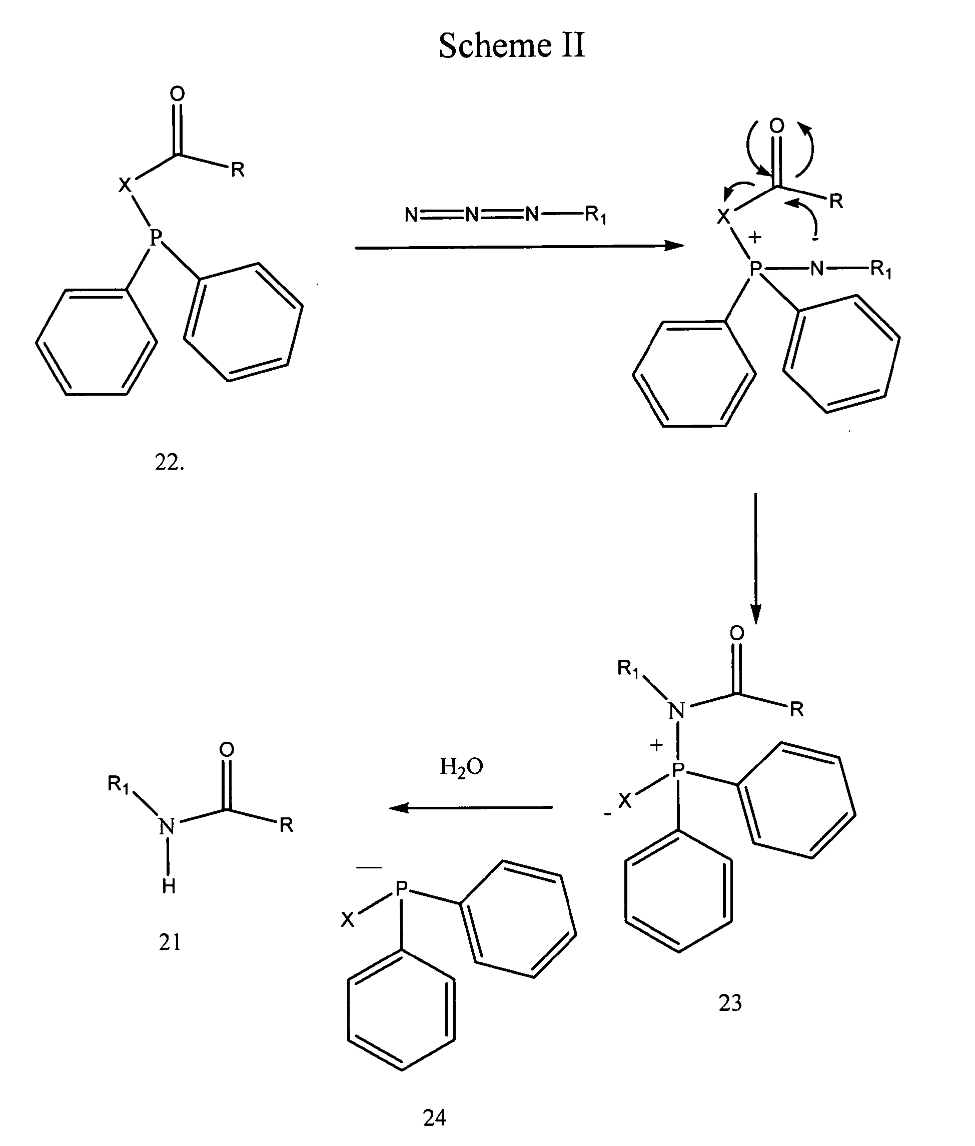

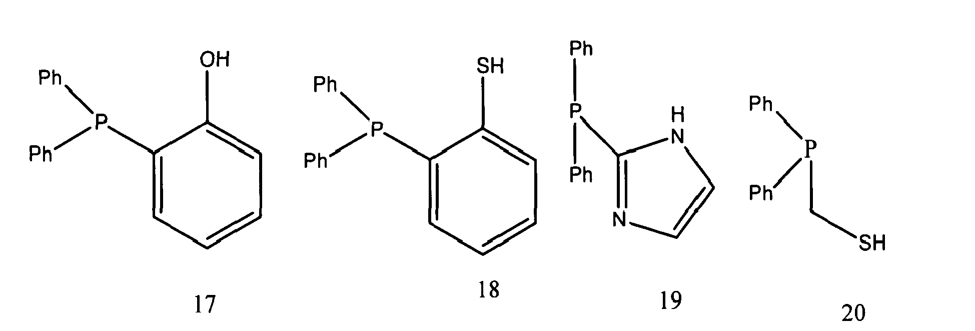

In this reaction, the phosphanes 17-20 are first acylated and then treated with the azide. The nucleophilic nitrogen atom of the aza-ylide then attacks the carbonyl group to cleave the linkage with the phosphonium species. Hydrolysis of the rearranged product 23 produces the amide 21 and liberates the phosphane oxide 24. See Köhn et al.

Thus, the Staudinger ligation is known to be an efficient method for the preparation of bioconjugates. See, e.g., Breinbaur, R; et al, *supra,* and references cited therein, incorporated herein by reference. Several azido-modified nucleotides and nucleosides have been synthesized or are commercially available. These include 2-azidodeoxyadenosine (Sekine, M; et al., *Tet. Lett.,* **2001**, 42, 9215-9219, and references cited therein), 2'-azido-2'-deoxynucleoside-5'-triphosphates (Commercially available from TriLink Biotechnologies) and 5'-β and 5'-γ phosphoazidates (Ofengand, J.; et al., *Biochem.,* **1977,** 16, 4312-4319). 2-azidoadenosine has been shown to undergo Staudinger chemistry (Sekine et al., *supra).* Azidophosphodiesters have been shown to undergo Staudinger chemistry (Gilyarov, V. A., *Inst. Elementorg. Soedin. Im. Nesmeyanova* 1990, 2, 465-468).

Reverse transcriptases, DNA polymerases, RNA polymerases and their mutants can incorporate certain modified dNTPs or rNTPs to some extent (Kukhanova, M.; et al., *Biochemica et Biophysica Acta,* 1986, 868, 136-144; Sousa, R.; Padilla, R., et al. *Nucleic Acids Research,* 2002, Vol. 30, No. 24 **e138;** Khorana, H. G.; et al., *J Biol. Chem.* **1972,** 247, 6140-6148; Goeff, S.P.; et al., *Proc. Natl. Aca. Sci. USA* **1997,** 94, 407-41; Suzuki, M.; et al., *Mutation Research* **2001, 485,** 197-207), each of which are incorporated herein by reference for all purposes.

For example, Padilla et al. reports on the incorporation of azido nucleotides with mutants of T7 RNA polymerase. With the wild-type enzyme, run-off transcription was reduced 15- and 50-fold in reactions with 2'-azidoUTP or 2'-azidoCTP, respectively. Run off transcription was undetectable in reactions with both 2'-azidoUTP and 2'-azidoCTP. With the mutant Y639F, use of 2'-azidoUTP or 2'-azidoCTP reduced run-off transcription by 70% as compared to reactions with four NTPs, while use of both 2'-azidoUTP and 2'-azidoCTP reduced run-off transcription by 11-fold (Fig. 2B, lane 8). With the double T7 mutant Y639F/H784A, use of a single azido-modified NTP reduced run-off transcription by only 40%, while use of two 2'-azidoNTPs reduced it by 3-fold.

In accordance with an aspect of the present invention, azido-modified nucleotide derivatives represented by the formula 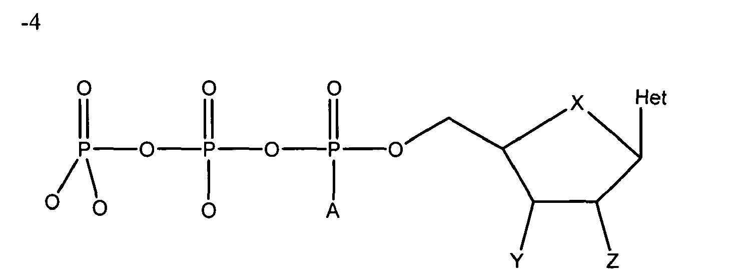 wherein A is O or N₃; X is O, S, NR₁ or CHR₂, wherein R₁ and R₂ are, independently, H, alkyl or aryl; Y is OH; Z is H, N₃, F or OR₁₀, wherein R₁₀ is H, alkyl or aryl, X is O, S, NR₁ or CHR₂, wherein R₁ and R₂ are, independently, H, alkyl or aryl; and Het is a heterocyclic group which is a cyclic moiety containing both carbon and a heteroatom,
wherein the heterocyclic group is optionally substituted with N₃ and wherein at least one of A, Z and Het comprises N₃ may be used as a substrates for reverse transcriptase, DNA polymerase, RNA polymerase or mutants of these enzymes designed to incorporate such azides into nucleic acid polymers. In another aspect of the present invention, it is proposed that conditions be determined under which the azido-modified nucleoside derivative may be used as substrates for the various enzymes. The preparation of various azido nucleotides has been described. See, e.g., "Synthesis and Properties of Nucleoside 5'-Phosphoazidates Derived from Guanosine and Adenosine Nucleotides: Effect on Elongation Factors G and T Dependent Reactions," Chládek, S. et al., *Biochemistry,* Vol. 16, No. 19, pp. 4312-4319 (1977), incorporated herein by reference for all purposes.

In accordance with an aspect of the present invention, preferred embodiments of the instant invention are set forth below: 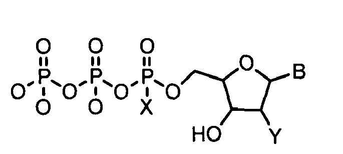 wherein B is selected from the group consisting of A, G, C, and T, X is N₃ or O, and Y is N₃ or H, provided that at least one of X or Y is N₃. Particularly preferred embodiments of this compound are as follows:
1. X is N₃ and Y is H.
2. X is O and Y is N₃.
   Other preferred compounds of the instant invention are as shown below 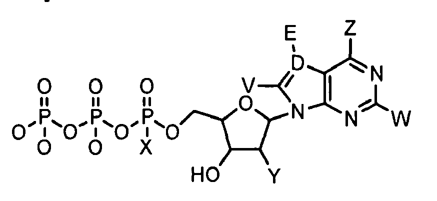 where X is O or N₃, V is H, -N₃ or L-N3, where L is a linker, D is C or N, E is H or pair of electrons or -L-N₃, where L is a linker, provided that when E is -L-N₃, D must be C, Z is NH₂ or OH and W is NH₂ or H. Particularly preferred embodiments of this aspect of the present invention are as set forth below:
3. V is H, E is -L-N₃, D is C, Z is NH₂ and W is H.
4. V is H, E is -L-N₃, D is C, Z is OH and W is NH₂
5. V is H, D is N, Z is NH₂ and W is N₃.
6. V is H, D is N, Z is OH and W is N₃.
7. V is -L-N₃, D is N, Z is OH and W is NH₂.
8. V is -L-N₃, D is N, Z is NH₂ and W is H.

Other embodiments of the instant invention are as shown below: 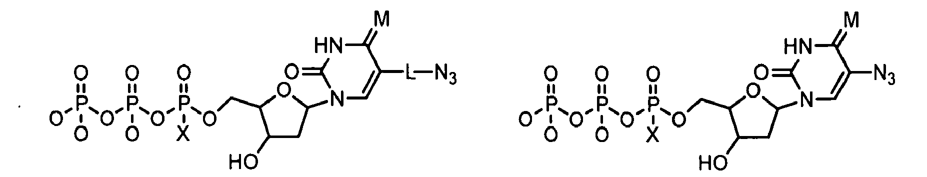 wherein X is O or N₃, M is NH or O, and L is a linker or bond. In particularly preferred embodiments of the instant invention, M is O and or NH. In accordance with an aspect of the present invention, an azido-modified nucleotide derivative incorporated into a polynucleotide is reacted with a compound bearing a detectable label. According to the present invention, the compound bearing the detectable label should be one with an entity that will react specifically with the azide moiety. Phosphane reagents were discussed above. In accordance with the present invention, a detectable moiety is coupled through a linker to a phosphane having a nearby acetyl group. In accordance with the present invention, two generic compounds and their preferred specific compounds 11 and 12 are shown below:
The first generic compound is 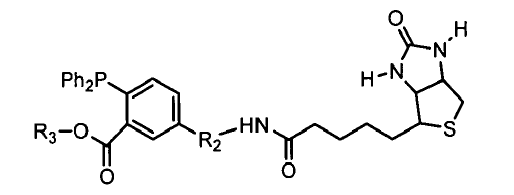 wherein R₂ is a linker, R₃ is selected from the group consisting of methyl, ethyl, propyl, and iso-propyl, preferably R3 is methyl; and preferably the linker is an alkyl amide linker. 11 is a preferred embodiment of the above phosphane. 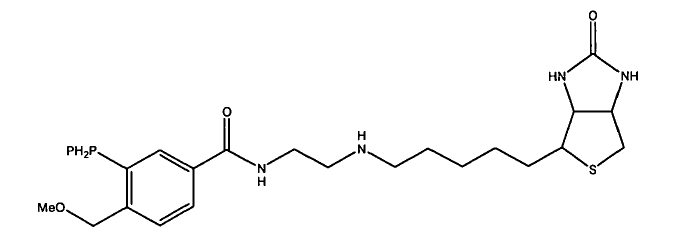
The other generic phosphane compound is 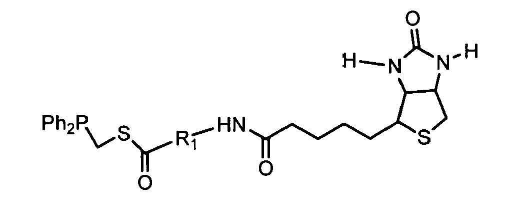 wherein R1 is a linker, preferably an alkyl linker and R3 is a linker, preferably having a sulfur atom adjacent to the carbonyl group. 12 is a preferred embodiment of the above phosphane.

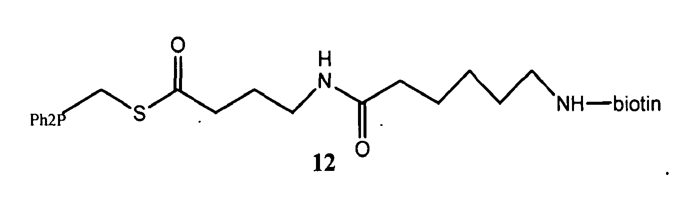

The azido group can then undergo Staudinger ligation with an appropriate phosphane possessing a reporter group, for example, biotin-phosphanes 11 and 12. This then results in attachment of a label through an amide linkage to either the phosphate or sugar backbone or on the heterocyclic base, see example below:

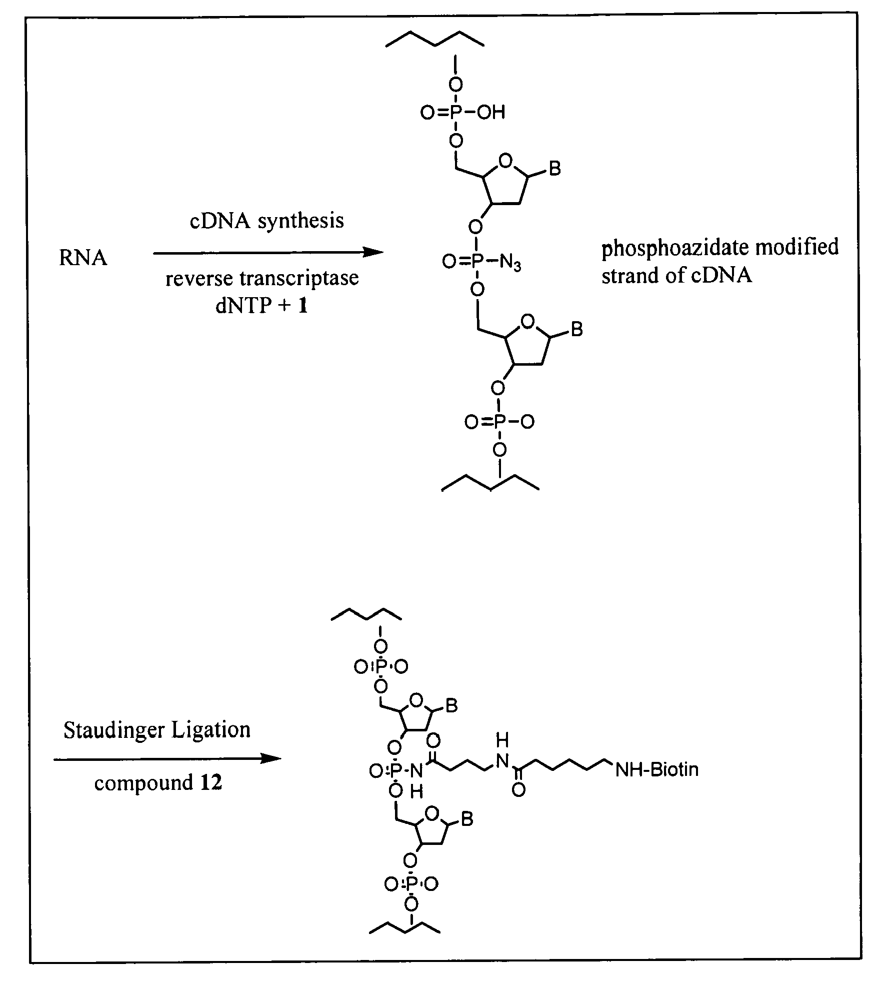

In accordance with an aspect of the present invention, the above biotin labeled cDNA can be hybridized to a nucleic acid array to determine the presence or absence of an RNA of interest.

In accordance with another aspect of the instant invention, another chemistry which can be used to link a detectable moiety to an azido group is termed "click" chemistry. Click chemistry involves the reaction of an azido group with an alkyne group linked to a detectable group. A preferred embodiment of instant invention is shown below: 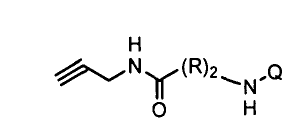 wherein R is a linker and Q is a detectable moiety. Preferably, Q is biotin and R is a water soluble linker having the structure (CH₂CHO)₃CH₂ and Q is biotin.

More preferably, the Click compound has the structure

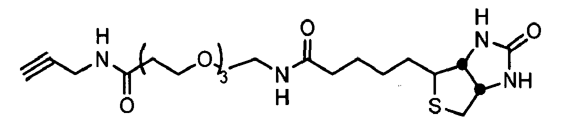

The alkyne group reacts with the azide group to give a triazole as follows: 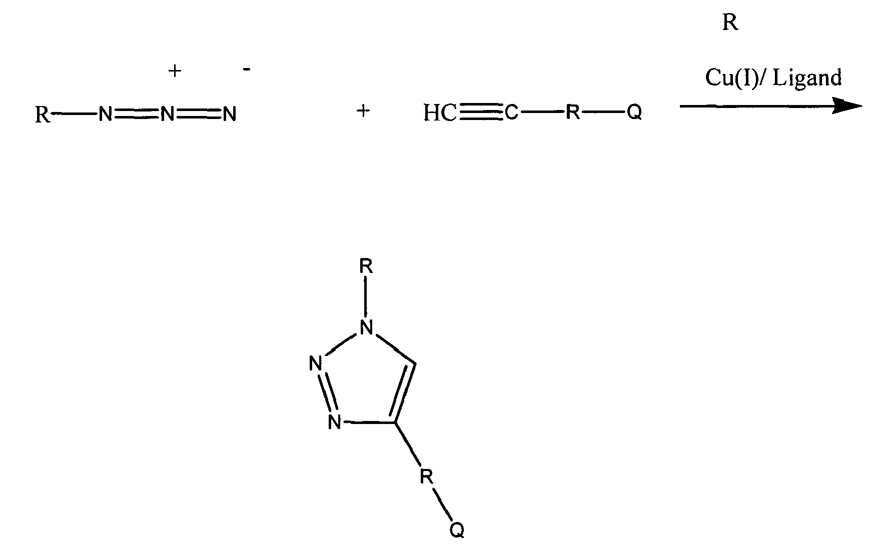 where R is a water soluble linker having the structure (CH₂CHO)₃CH₂ and Q is biotin. See, e.g., Agard, N, J, et al, J. Am. Chem. Soc. **2004**, 126, 15046-15047; Seo, T. S., et. al., Proc. Natl. Acad. Sci. USA 5488-5493, vol. 101, no. 15 (2004).

The Click reaction can be used to incorporate alkyne labeled detectable marker onto a nucleotide bearing an azide group as disclosed in accordance with an aspect of the instant invention. In accordance with an aspect of the present invention, when the azido modified nucleotide has been incorporated into a polynucleotide and functionalized via the Click reaction with a detectable moiety, the labeled polynucleotides hybridized to a nucleic acid array

Random oligomer primers for use in the present invention can be custom made, "off the shelf" or "home" made. The primers can be from about 6 to about 15 nucleotides in length. The amount of primer used will affect efficiency and the length of synthesized products. The range of weight ratios of hexamer to initial RNA input should be between about 1:100 and 10:1, preferably about 1:10. Higher ratios tend to yield shorter products. Enzymes which can be used to synthesize second strand cDNA are any known in the art for such purpose. *E*. *coli* DNA polymerase I can be used, as well as Klenow fragment. These can optionally be used with DNA ligase which will promote longer products.

Reverse transcription is performed in the method of the invention according to standard techniques known in the art. The reaction is typically catalyzed by an enzyme from a retrovirus, which is competent to synthesize DNA from an RNA template. According to the present method, the primer used for reverse transcription has two parts: one part for annealing to the RNA molecules in the cell sample through complementarity and a second part comprising a strong promoter sequence. Typically the strong promoter is from a bacteriophage, such as SP6, T7 or T3. Promoters which drive robust in vitro transcription are desirable. Because most populations of MRNA from biological samples do not share any sequence homology other than a poly(dA) tract at the 3' end, the first part of the primer typically comprises a poly(dT) sequence which is generally complementary to most mRNA species. The length of the tract is typically from about 5 to 20 nucleotides, more preferably about 10 to 15 nucleotides. Alternatively, if a subpopulation of RNA is desired, a primer which is complementary to a common sequence feature in the subpopulation can be used. Yet another type of priming employs random oligomers. Such oligomers should yield a full and representative set of cDNA. The orientation of the promoter sequence is important. It is typically at the 5' end of the primer, so that the 3' end can successfully anneal and drive reverse transcription. Moreover, the promoter sequence is oriented in such a fashion that it is "opposite" the 3' end of the MRNA. Thus upon second strand synthesis, the double stranded promoter will be at the 3' end of the gene, in an orientation favorable for producing reverse strand (negative strand, or antisense) RNA. This orientation is termed "antisense" orientation. Hybrids of first strand cDNA and MRNA can be denatured according to any method known in the art. These include the use of heat and the use of alkali. Heat treatment is the preferred method. Denaturation is desirable until less than 50% of the hybrids remain annealed. More denaturation is desirable, such as until less than 75%, 85% or 95% of the hybrids remain annealed as hybrids.

Transcription of the double stranded cDNA molecules is a linear process which creates large amounts of product from small input amount, without greatly distorting the relative amounts of input. Thus the transcription process while being efficient is "linear" rather than "exponential." Labeled ribonucleotides can be used during transcription of the double stranded cDNA. These can be radioactively labeled, with such isotopes as 32P, 3H, and 32S. Fluorescently labeled ribonucleotides can also be used. Biotin labeled nucleotides can also be used. Subsequent to incorporation, labeled avidin can be bound to biotin-labeled polynucleotides. The labeled avidin can contain any desirable and convenient detectable label.

Quantitation of particular RNA molecules within the population of copy RNA can be done according to any means known in the art. These include but are not limited to Northern blotting and hybridization to nucleic acid arrays. Typically, some sort of hybridization step must be involved to provide the specificity required to measure transcripts individually. Alternatively, the cRNA can be reverse transcribed into cDNA and a specific cDNA species can be amplified to obtain specificity. Copy RNA can be used for any use known in the art, not merely quantitation. It can be used for cloning, and/or expression, or as a probe. Such uses can be applied to determining a diagnosis or prognosis, to determining an etiological basis for disease, for determining a cell type or species source, for identifying infectious organisms in foods, hospitals, ventilation systems, and for testing drugs for their main or side effects. Other applications will be readily apparent to those of skill in the art.

Detectable labels suitable for use in the present invention include any composition which can be modified to be coupled to an azido group and detectable by spectroscopic, photochemical, biochemical, immunochemical, electrical, optical or chemical means. Useful labels in the present invention include biotin for staining with labeled streptavidin conjugate, magnetic beads (e.g., DynabeadsTM), fluorescent dyes (e.g., fluorescein, texas red, rhodamine, green fluorescent protein, and the like), radiolabels (e.g., .sup.3 H, .sup.125 I, .sup.35 S, .sup.14 C, or .sup.32 P), enzymes (e.g., horse radish peroxidase, alkaline phosphatase and others commonly used in an ELISA), and colorimetric labels such as colloidal gold or colored glass or plastic (e.g., polystyrene, polypropylene, latex, etc.) beads. Patents teaching the use of such labels include U.S. Pat. Nos. 3,817,837; 3,850,752; 3,939,350; 3,996,345; 4,277,437; 4,275,149; and 4,366,241.

Means of detecting such labels are well known to those of skill in the art. Thus, for example, radiolabels may be detected using photographic film or scintillation counters, fluorescent markers may be detected using a photodetector to detect emitted light. Enzymatic labels are typically detected by providing the enzyme with a substrate and detecting the reaction product produced by the action of the enzyme on the substrate, and colorimetric labels are detected by simply visualizing the colored label.

The label may be added to the target (sample) nucleic acid(s) prior to, or after the hybridization. So called "direct labels" are detectable labels that are directly attached to or incorporated into the target (sample) nucleic acid prior to hybridization. In contrast, so called "indirect labels" are joined to the hybrid duplex after hybridization. Often, the indirect label is attached to a binding moiety that has been attached to the target nucleic acid prior to the hybridization. Thus, for example, the target nucleic acid may be biotinylated before the hybridization. After hybridization, an aviden-conjugated fluorophore will bind the biotin bearing hybrid duplexes providing a label that is easily detected. For a detailed review of methods of labeling nucleic acids and detecting labeled hybridized nucleic acids see Laboratory Techniques in Biochemistry and Molecular Biology, Vol. 24: Hybridization With Nucleic Acid Probes, P. Tijssen, ed. Elsevier, N.Y., (1993)).

A nucleic acid array according to the present invention is any solid support having a plurality of different nucleotide sequences attached thereto or associated therewith. One preferred type of nucleic acid array that is useful in the present invention include those that are commercially available from Affymetrix (Santa Clara, CA) under the brand name GeneChip®. Example arrays are shown on the website at affymetrix.com.

GeneChip Analysis.

GeneChip® nucleic acid probe arrays are manufactured using technology that combines photolithographic methods and combinatorial chemistry. In a preferred embodiment, over 280,000 different oligonucleotide probes are synthesized in a 1.28 cm x 1.28 cm area on each array. Each probe type is located in a specific area on the probe array called a probe cell. Measuring approximately 24 µm x 24 µm, each probe cell contains more than 10⁷ copies of a given oligonucleotide probe.

Probe arrays are manufactured in a series of cycles. A glass substrate is coated with linkers containing photolabile protecting groups. Then, a mask is applied that exposes selected portions of the probe array to ultraviolet light. Illumination removes the photolabile protecting groups enabling selective nucleotide phosphoramidite addition only at the previously exposed sites. Next, a different mask is applied and the cycle of illumination and chemical coupling is performed again. By repeating this cycle, a specific set of oligonucleotide probes is synthesized, with each probe type in a known physical location. The completed probe arrays are packaged into cartridges.

In accordance with an aspect of the present invention, a method is presented for detecting the presence or absence of a mRNA in a nucleic acid sample by hybridization to a nucleic acid array, the method comprising the steps of providing a nucleic acid sample comprising mRNA; hybridizing the mRNA with an oligonucleotide primer comprising an oligonucleotide homologous to said mRNA; providing a 2'-deoxynucleotide triphosphate derivative having an azido group allowing for the chemical attachment of a phosphone derivatized detectable label; reverse transcribing said mRNA with a reverse capable of incorporating the deoxynucleotide derivative with a rate and fidelity substantially similar to that for natural 2' deoxynucleotide triphosphates to provide reverse transcribed DNA homologous to all or part of said mRNA having azido groups; reacting the azido groups on the DNA with a phosphone derivatized detectable label to provide labeled DNA; and hybridizing the labeled DNA to said nucleic acid array to detect the presence or absence of the mRNA.

Reverse transcription is performed in the method of the invention according to standard techniques known in the art. The reaction is typically catalyzed by an enzyme from a retrovirus, which is competent to synthesize DNA from an RNA template. According to the present method, the primer used for reverse transcription has two parts: one part for annealing to the RNA molecules in the cell sample through complementarity and a second part comprising a strong promoter sequence. Typically the strong promoter is from a bacteriophage, such as SP6, T7 or T3. Promoters which drive robust in vitro transcription are desirable. Because most populations of MRNA from biological samples do not share any sequence homology other than a poly(da) tract at the 3' end, the first part of the primer typically comprises a poly(dT) sequence which is generally complementary to most mRNA species. The length of the tract is typically from about 5 to 20 nucleotides, more preferably about 10 to 15 nucleotides. Alternatively, if a subpopulation of RNA is desired, a primer which is complementary to a common sequence feature in the subpopulation can be used. Yet another type of priming employs primer oligomers. Such oligomers should yield a full and representative set of cDNA. The orientation of the promoter sequence is important. It is typically at the 5' end of the primer, so that the 3' end can successfully anneal and drive reverse transcription. Moreover, the promoter sequence is oriented in such a fashion that it is "opposite" the 3' end of the MRNA. Thus upon second strand synthesis, the double stranded promoter will be at the 3' end of the gene, in an orientation favorable for producing reverse strand (negative strand, or antisense) RNA. This orientation is termed "antisense" orientation. Hybrids of first strand cDNA and MRNA can be denatured according to any method known in the art. These include the use of heat and the use of alkali. Heat treatment is the preferred method. Denaturation is desirable until less than 50% of the hybrids remain annealed. More denaturation is desirable, such as until less than 75%, 85% or 95% of the hybrids remain annealed as hybrids.

Transcription of the double stranded cDNA molecules is a linear process which creates large amounts of product from small input amount, without greatly distorting the relative amounts of input. Thus the transcription process while being efficient is "linear" rather than "exponential." Labeled ribonucleotides can be used during transcription of the double stranded cDNA. These can be radioactively labeled, with such isotopes as 32P, 3H, and 32S. Alternatively, in accordance with the present invention, nucleotides can be modified to bear an azido functionality. After reverse transcription or at subsequent down stream steps, azido labeled nucleotides can be reacted with detectable moieties via the click reaction or with phosphanes as discussed above. Fluorescently labeled ribonucleotides can also be used. Biotin labeled nucleotides can also be used. Subsequent to incorporation, labeled avidin can be bound to biotin-labeled polynucleotides.

The labeled avidin can contain any desirable and convenient detectable label. Quantitation of particular RNA molecules within the population of copy RNA can be done according to any means known in the art. These include but are not limited to Northern blotting and hybridization to nucleic acid arrays. Typically, some sort of hybridization step must be involved to provide the specificity required to measure transcripts individually. Alternatively, the cRNA can be reverse transcribed into cDNA and a specific cDNA species can be amplified to obtain specificity. Copy RNA can be used for any use known in the art, not merely quantitation. It can be used for cloning, and/or expression, or as a probe. Such uses can be applied to determining a diagnosis or prognosis, to determining an etiological basis for disease, for determining a cell type or species source, for identifying infectious organisms in foods, hospitals, ventilation systems, and for testing drugs for their main or side effects. Other applications will be readily apparent to those of skill in the art.

A primer is a single-stranded oligonucleotide capable of acting as a point of initiation for template-directed DNA synthesis under suitable conditions e.g., buffer and temperature, in the presence of four different nucleoside triphosphates and an agent for polymerization, such as, for example, DNA or RNA polymerase or reverse transcriptase. The length of the primer, in any given case, depends on, for example, the intended use of the primer, and generally ranges from 15 to 30 nucleotides. Short primer molecules generally require cooler temperatures to form sufficiently stable hybrid complexes with the template. A primer need not reflect the exact sequence of the template but must be sufficiently complementary to hybridize with such template. The primer site is the area of the template to which a primer hybridizes. The primer pair is a set of primers including a 5' upstream primer that hybridizes with the 5' end of the sequence to be amplified and a 3 ' downstream primer that hybridizes with the complement of the 3' end of the sequence to be amplified.

The requirement that the reverse transcriptase be capable of incorporating the deoxynucleotide derivative into a growing DNA strand with a rate and fidelity substantially similar to that for natural 2' deoxynucleotide triphosphates is both a flexible and a practical one. The key to this requirement is that the enzyme/substrate combination provide a workable labeling system, considering the rate of incorporation and the fidelity of incorporation, i.e. that the template be copied with a relatively small number of errors. In this regard, for example, a G or G analog should be incorporated by the reverse transcriptase when a C is presented on the mRNA template. Also, the rate of the reaction must be kept so that the assay can be carried out in a reasonable period of time, e.g., a total time of 24-48 hours.

In order to meet these requirements, persons of skill in the art can modify the enzyme to accept different substrates, for example by deleting or changing amino acids in the enzyme. In addition, azido substrates can be modified in a number of ways so that they work more efficiently and with greater fidelity with available wild type or mutant enzymes. Searching for variants in the enzymes and substrates to identify optimal combinations is within the ambit of those of skill in the art without undue experimentation.

This requirement is applicable to all the enzyme-substrate combinations claimed with respect to an aspect of the instant invention. Thus, DNA polymerases, RNA polymerases, ligases and terminal transferases must all be matched with the appropriate azido substrates. Obviously, fidelity is not an issue for enzymes which label the terminal ends of nucleic acids, but reaction rates are just as important as for the polymerases.

One preferred embodiment in regard to azido nucleotide derivatives has the structure: 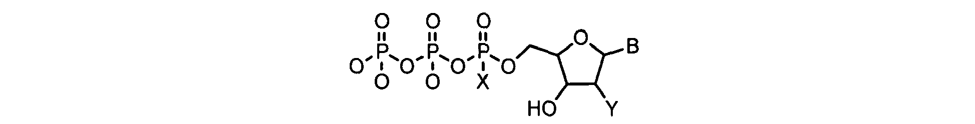 where B is selected from the group consisting of A, G, C, T and derivatives thereof, X is
O or N₃ and Y is H or N₃ and at least one of X and Y is N₃.

Preferred phosphane derivatized detectable label embodiments have the following structures: 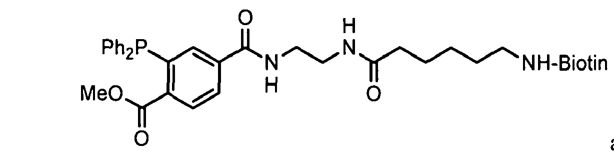 and

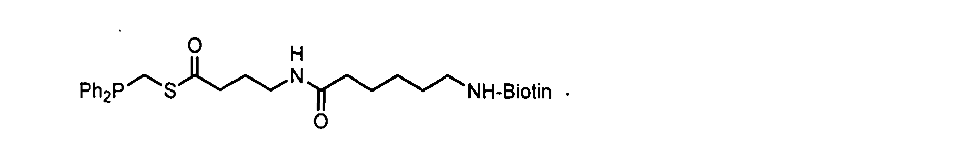

Other preferred embodiments of azido nucleotide derivative are as follows: 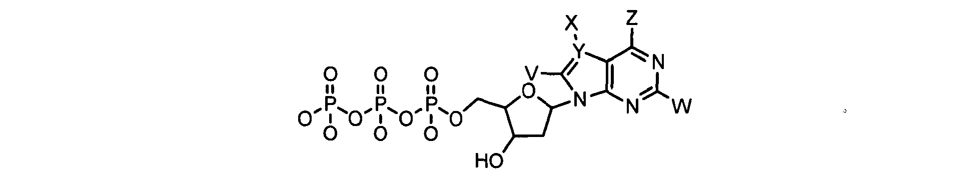 wherein V is H, X is - N₃ or -R- N₃, wherein R is a linker, Y is C, Z is NH₂, and W is H; 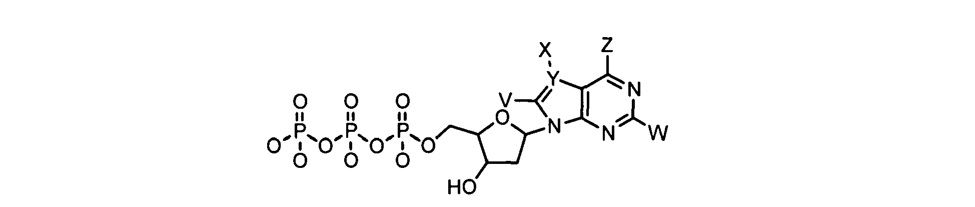 wherein V is H, X is - N₃ or -R- N₃ wherein R is a linker, Y is C, Z is OH, W is NH₂; 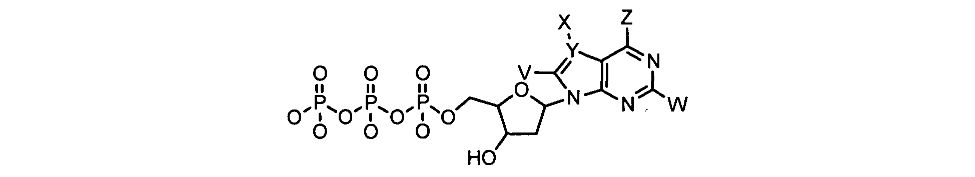 wherein V is H, Y is N, Z is NH₂, and W is N₃; 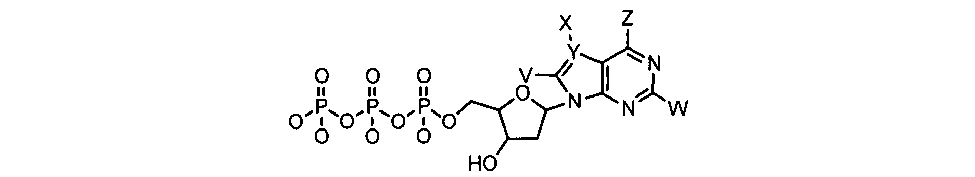 wherein V = H, Y = N, Z = OH, W = N₃; 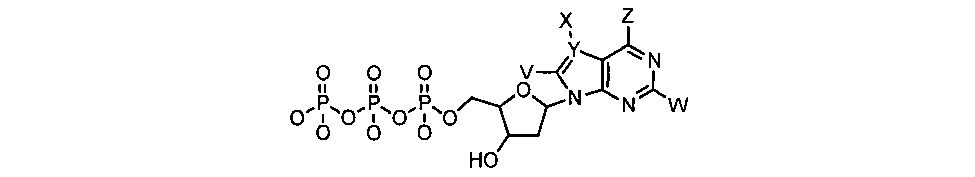 wherein V is - N₃ or -R- N₃ wherein R is a linker, Y is N, Z is OH, and W is NH₂; 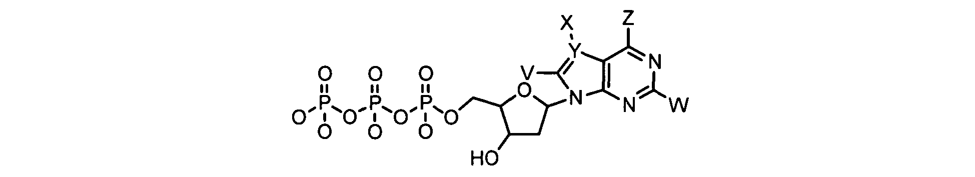 wherein V is - N₃ or -R- N₃ wherein R is a linker, Y is N, Z is NH₂, and W is H; and 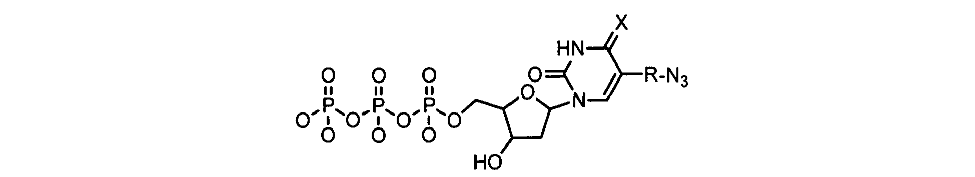 wherein X is NH or O and R is a linker or a bond.

In a particularly preferred embodiment of the method involving reverse transcriptase, the azido nucleotide derivative has the structure 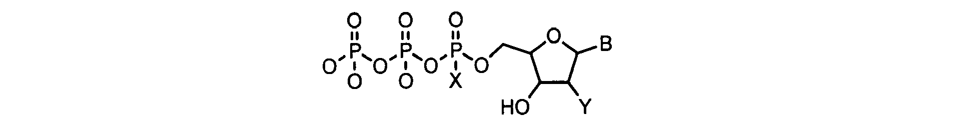 wherein B is selected from the group consisting of A, G, C, T and derivatives thereof, X is O or N₃ and Y is O or N₃ and at least one of X and Y is N₃ and said phosphone derivatized detectable label has the structure selected from the group consisting of: 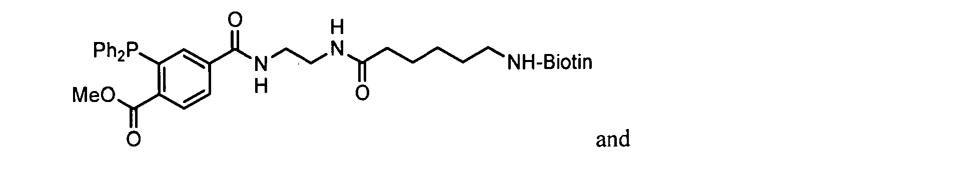 and

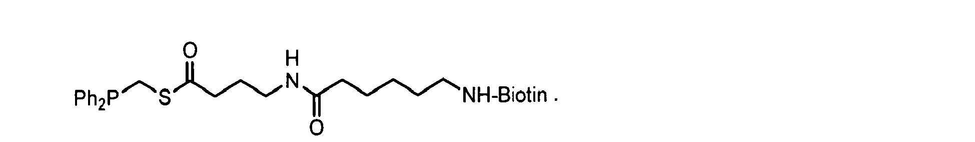

More preferable the nucleotide derivative has the structure: 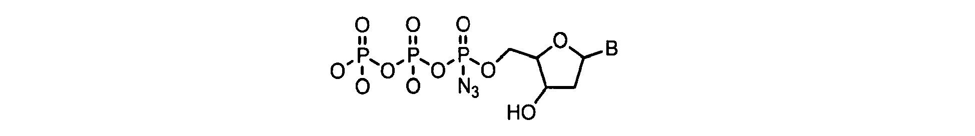 wherein B is selected from the group consisting of A, G, C, T, and the phosphone derivatized detectable label has the structure:

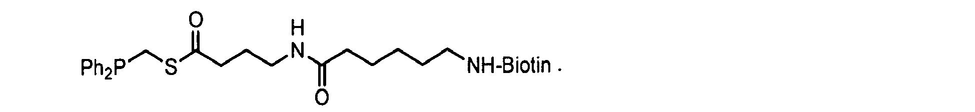

Preferably, the labeled DNA has the structure:

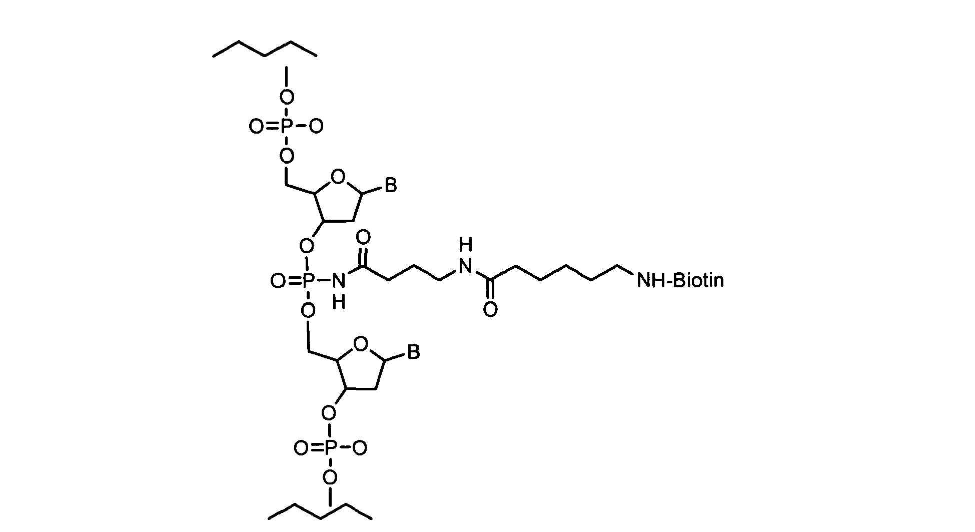

According to one aspect of the present invention, a method is presented for detecting the presence or absence of a mRNA in a nucleic acid sample by hybridization to a nucleic acid array, the method has the steps of providing a nucleic acid sample of mRNA; hybridizing the mRNA with an oligonucleotide probe homologous to a portion of the mRNA; providing a 2'-deoxynucleotide triphosphate derivative having an orthogonal group allowing for the specific chemical attachment of a derivatized detectable label; reverse transcribing said mRNA with a reverse transcriptase to provide reverse transcribed DNA homologous to all or part of said mRNA comprising one or more reactive orthogonal groups; reacting the orthogonal groups on the DNA with a derivatized detectable label to provide labeled DNA; and hybridizing the labeled DNA to said nucleic acid array to detect the presence or absence of the mRNA. Preferably, the reactive orthogonal group is an azido group. It is also preferred that the oligonucleotide probe has a poly dT sequence which can hybridize to the poly A tail of eukaryotic mRNA. The oligonucleotide probe is preferably from 12-18 nucleotides in length.

In another preferred embodiment of the instant invention, the step of hybridizing the mRNA with a primer comprising an oligonucleotide is carried out by hybridizing the mRNA with a plurality of random primers at least one of which said random primers is homologous to a portion of said mRNA and hybridizes to said mRNA. Preferably, the random primer is from 6-12 nucleotides in length. More preferably, the random primer is from 6-9 nucleotides in length. Still more preferably the random primers are 8 nucleotides.

The 2'-deoxynucleotide triphosphate derivative having an azido group preferably have the structure 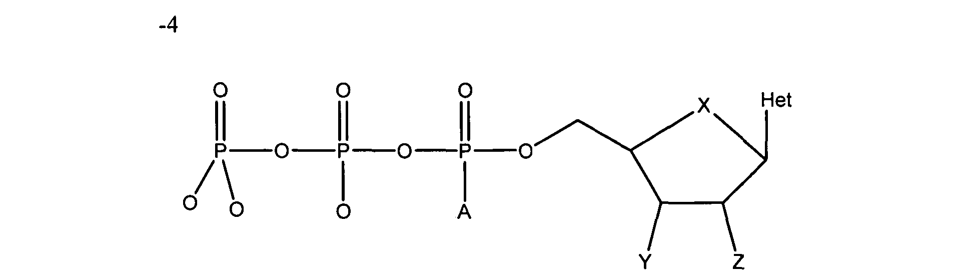 wherein A is O or N₃; X is O, S, NR₁ or CHR₂, wherein R₁ and R₂ are, independently, H, alkyl or aryl; Y is OH; Z is H, N₃, F or OR₁₀, wherein R₁₀ is H, alkyl or aryl, X is O, S, NR₁ or CHR₂, wherein R₁ and R₂ are, independently, H, alkyl or aryl; and Het is a heterocyclic group which is a cyclic moiety containing both carbon and a heteroatom, wherein the heterocyclic group is optionally substituted with N₃ and wherein at least one of A, Z and Het comprises N₃.

More preferably, the 2'-deoxynucleotide triphosphate derivative having an azido group has the structure: 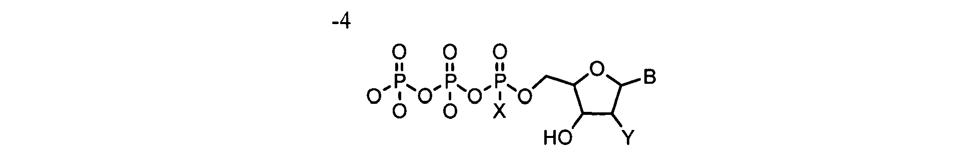 wherein B is selected from the group consisting of A, G, C, T and derivatives thereof, X is O or N₃ and Y is H or N₃ and at least one of X and Y is N₃.

Preferably, the derivatized detectable label is a phosphone or a click derivative. More preferably, the derivatized detectable label is a phosphone having the structure 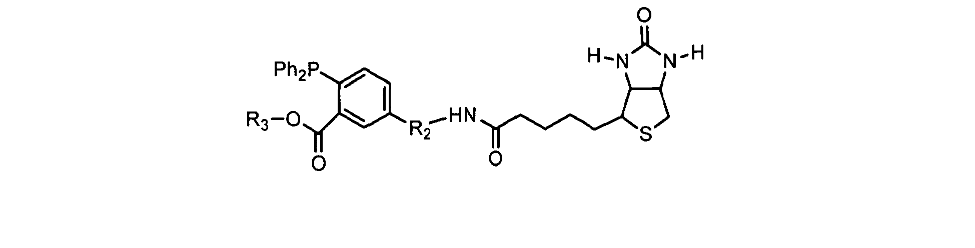 wherein R₂ is a linker, and R₃ is selected from the group consisting of methyl, ethyl, propyl, and iso-propyl. More preferably, the phosphone has the structure:

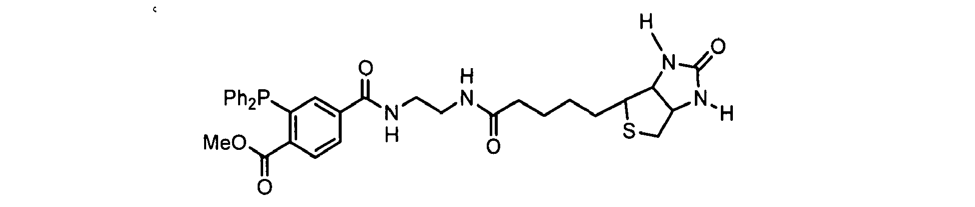

According to another aspect of the present invention, the derivatized detectable label is a phosphone having the structure 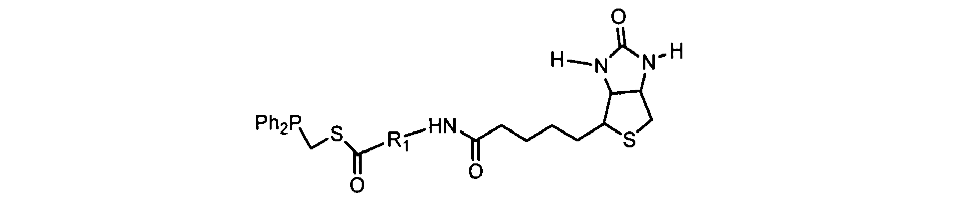 wherein R₁ is a linker and R₃ is a linker. More preferably, R₁ is an alkyl linker and R₃ is a linker having a sulfer atom adjacent to the carbonyl group. In more preferred embodiments the above phosphone has the structure:

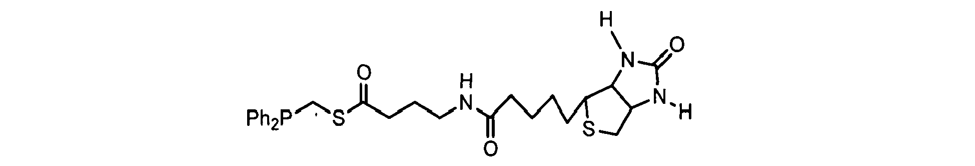

In another preferred embodiment of the instant invention, the derivatized detectable label is a click reagent having the structure

HC≡C-R-Q

wherein R is a linker and Q is a detectable moiety. More preferably, the click reagent has the structure:

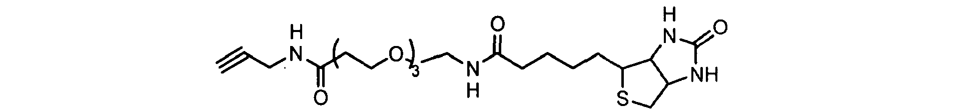

Purines are a particularly preferred 2'-deoxynucleotide triphosphate derivative having the structure: 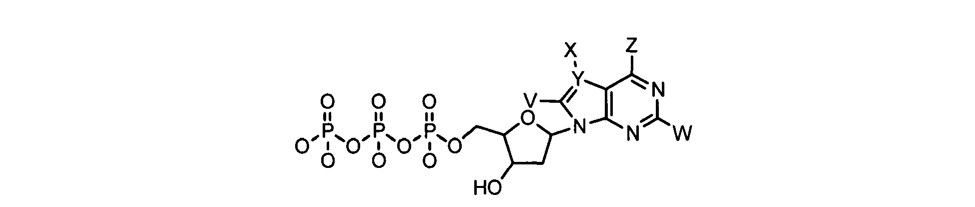 wherein V is H, X is -R-N₃, wherein R is a linker or a bond, Y is N or C, Z is OH, N₃ or
NH₂, and W is H, NH₂ or N₃, wherein at least one of X, Z or W is N₃.

Certain non-natural bases are preferred as 2'-deoxynucleotide triphosphate derivatives. The structure below is particularly preferred: 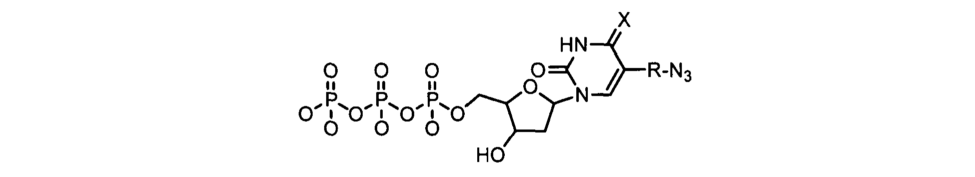 wherein X is NH or O and R is a linker or a bond.

In a particularly preferred embodiment of the instant invention, the 2'-deoxynucleotide triphosphate derivative has the structure 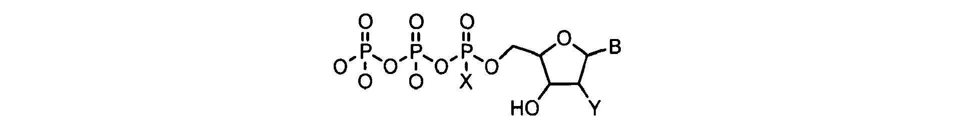 wherein B is selected from the group consisting of A, G, C, T and derivatives thereof, X is O or N₃ and Y is O or N₃ and at least one of X and Y is N₃ and the derivatized detectable label has the structure selected from the group consisting of: 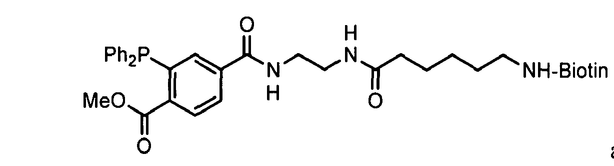 and

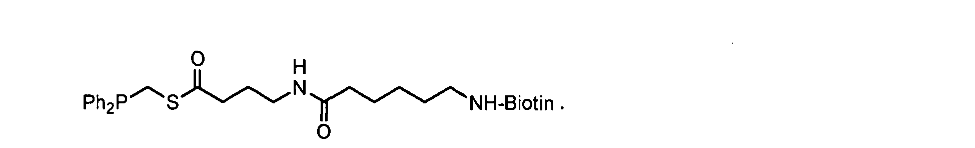

More preferably, the nucleotide derivative has the structure: 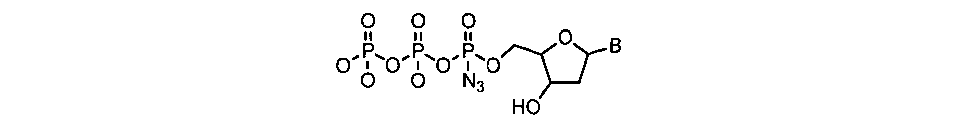 wherein B is selected from the group consisting of A, G, C, T, and the phosphone derivatized detectable label has the structure:

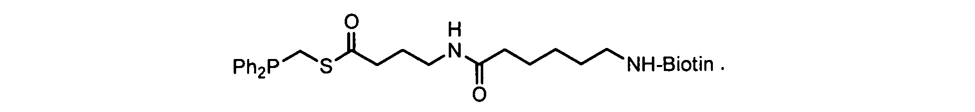

The labeled DNA of an aspect of the present invention, has the structure: 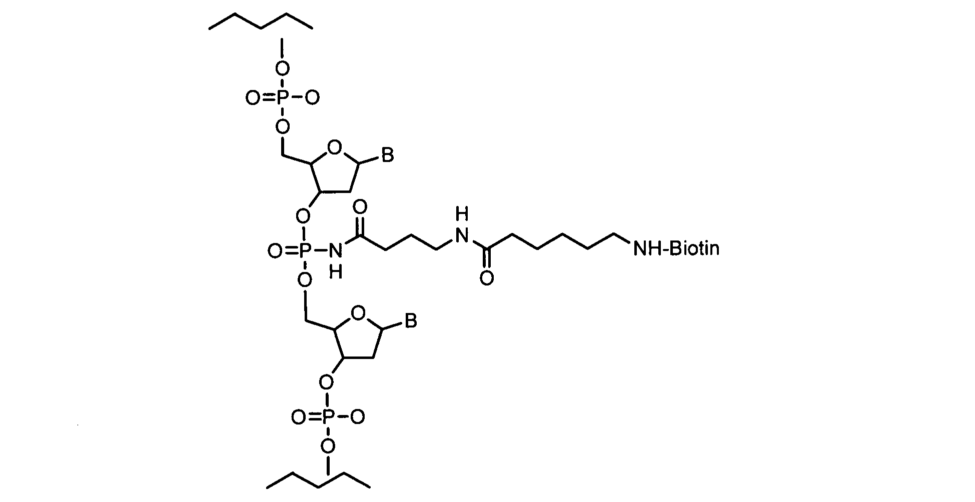 wherein B is a base selected from the group consisting of A, G, T and C

According to another aspect of the present invention, a method for detecting the presence or absence of a mRNA in a nucleic acid sample by hybridization to a nucleic acid array, the method comprising the steps of providing a nucleic acid sample comprising mRNA; hybridizing the mRNA with an oligonucleotide probe comprising a poly dT sequence and a T7 RNA polymerase promoter; reverse transcribing the mRNA to provide single stranded DNA; converting the single stranded DNA to double stranded DNA wherein said T7 RNA polymerase promoter is oriented to provide cRNA; providing a ribonucleotide triphosphate having an orthogonal reactive group which may be incorporated into an RNA strand by a native or mutant T7 RNA polymerase; transcribing said double stranded DNA with a natural or mutant T7 RNA polymerase with said ribonucleotide triphosphate having said orthogonal reactive group to provide cRNA having orthogonal reactive groups; reacting said orthogonal reactive groups on said cRNA with a derivatized detectable label to provide labeled cRNA; and hybridizing said labeled cRNA to said nucleic acid array to detect the presence or absence of said mRNA.

Preferably, the T7 RNA polymerase is natural. Alternatively, the T7 RNA polymerase is a mutant. More preferably, the mutant is Y639F/H784A. Preferably, the orthogonal reactive group comprises an azido group.

More preferably, the ribonucleotide triphosphate is selected from the group consisting of 2'-azidoUTP or 2'-azidoCTP.

According to another aspect of the present invention, a method for detecting the presence or absence of a polynucleotide of interest on a nucleic acid array is presented, said method having the steps of providing a nucleic acid sample comprising a polynucleotide; providing a nucleotide triphosphate having a reactive orthogonal group; enzymatically amplifying the polynucleotide with the nucleotide triphosphate to provide amplified nucleic acids having orthogonal reactive groups; reacting said orthogonal groups on said nucleic acids with a detectable label to provide labeled nucleic acids; hybridizing said labeled nucleic acids to a nucleic acid array; and detecting the presence or absence of said polynucleotide.

Preferably, the polynucleotide comprises genomic DNA. The polynucleotide is also preferably selected from the group consisting of mitochondrial DNA, RNA, and mRNA.

The enzyme is preferably selected from the group consisting of an RNA polymerase, a DNA polymerase and a reverse transcriptase.

The orthogonal group preferably comprises an azido group. The azido group nucleoside triphosphate is selected from the group consisting of: 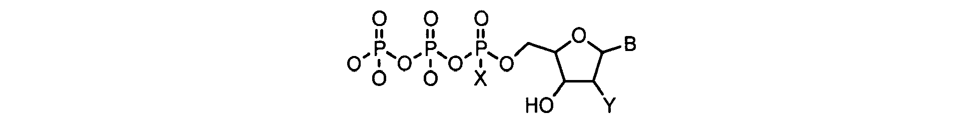 where B is selected from the group of bases consisting of A, G, C, T and derivatives thereof, X is O or N₃ and Y is O or N₃ and at least one of X and Y is N₃; 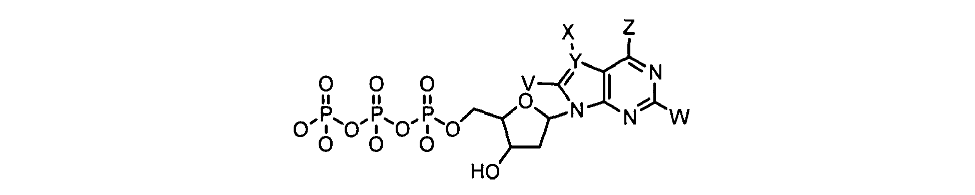 wherein V is H or N₃, X is-R- N₃, wherein R is a linker or a bond, Y is C or N, Z is NH₂, OH or N₃ and W is NH₂, N₃ or H, wherein at least one of V, X, Z or W is N₃; and 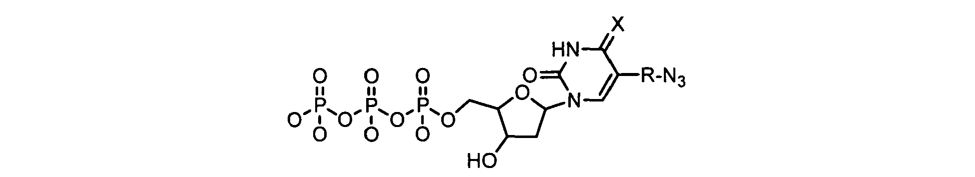 wherein X is NH or O and R is a linker or a bond.

The detectable label is preferably selected from the group consisting of 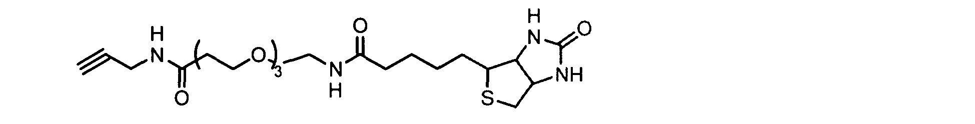 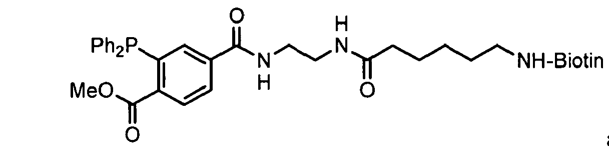 and

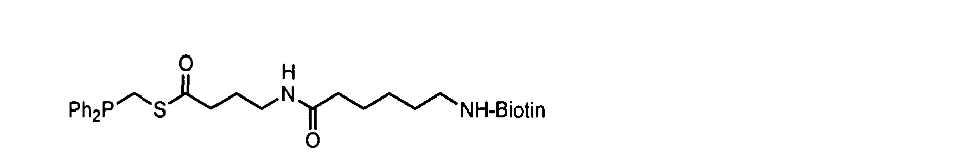

In a further aspect, the invention relates to use of a nucleotide triphosphate derivative having an orthogonal group, e.g. an azido group, allowing for the specific chemical attachment of a derivative detectable label, in detection of a polynucleotide, optionally in detection of a polynucleotide on a nucleic acid array.

In one embodiment, the nucleotide triphosphate derivative can be any one of the nucleotide triphosphate derivatives described herein.

In yet a further aspect, the invention relates to use of a derivatized detectable label and a nucleotide triphosphate derivative having an orthogonal group, e.g. an azido group, allowing for the specific chemical attachment of said derivatized detectable label, for detection of a polynucleotide, optionally for detection of a polynucleotide or a nucleic acid array.

In one embodiment, any one of the nucleotide triphosphate derivatives described herein can be used in combination with any one of the derivatized detectable labels described herein.

All patents, patent applications, and literature cited in the specification are hereby incorporated by reference in their entirety.

The invention is intended, as is made clear from the content of the specification to cover all subject matter and all combinations of subject matter described herein. However, the skilled person will understand that the invention differs from each and every relevant prior art disclosure and that the invention is intended to be delimited from the prior art and exclude any embodiment therein.

The invention has been described with reference to various specific and preferred embodiments and techniques. However, it should be understood that many variations and modifications may be made while remaining within the spirit and scope of the invention.

The following paragraphs form part of the description:
1. A method for detecting the presence or absence of a mRNA in a nucleic acid sample by hybridization to a nucleic acid array, said method comprising the steps of
   providing a nucleic acid sample comprising mRNA;
   hybridizing said mRNA with an oligonucleotide probe homologous to a portion of said mRNA;
   providing a 2'-deoxynucleotide triphosphate derivative having an orthogonal group allowing for the specific chemical attachment of a derivatized detectable label;
   reverse transcribing said mRNA with a reverse transcriptase to provide reverse transcribed DNA homologous to all or part of said mRNA comprising one or more reactive orthogonal groups;
   reacting said orthogonal groups on said DNA with a derivatized detectable label to provide labeled DNA; and
   hybridizing said labeled DNA to said nucleic acid array to detect the presence or absence of said mRNA.
2. A method according to paragraph 1 wherein said reactive orthogonal group is an azido group.
3. A method according to paragraph 1 wherein said oligonucleotide probe comprises a poly dT sequence.
4. A method according to paragraph 1 wherein said oligonucleotide probe is from 12-18 nucleotides in length.
5. A method according to paragraph 1 wherein said step of hybridizing said mRNA with a primer comprising an oligonucleotide is carried out by hybridizing said mRNA with a plurality of random primers at least one of which said random primers is homologous to a portion of said mRNA and hybridizes to said mRNA.
6. A method according to paragraph 5 wherein said random primer is from 6-12 nucleotides in length.
7. A method according to paragraph 5 wherein said random primer is from 6-9 nucleotides in length.
8. A method according to paragraph 6 wherein said random primer is 8 nucleotides.
9. A method according to paragraph 2 wherein said 2'-deoxynucleotide triphosphate derivative having an azido group 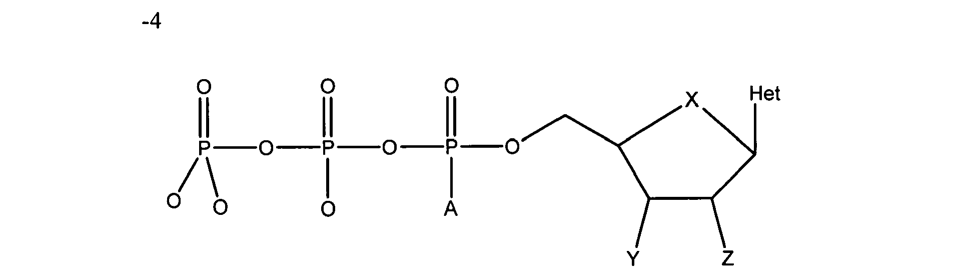 wherein A is O or N₃; X is O, S, NR₁ or CHR₂, wherein R₁ and R₂ are, independently, H, alkyl or aryl; Y is OH; Z is H, N₃, F or OR₁₀, wherein R₁₀ is H, alkyl or aryl, X is O, S, NR₁ or CHR₂, wherein R₁ and R₂ are, independently, H, alkyl or aryl; and Het is a heterocyclic group which is a cyclic moiety containing both carbon and a heteroatom,
   wherein the heterocyclic group is optionally substituted with N₃ and wherein at least one of A, Z and Het comprises N₃.
10. A method according to paragraph 2 wherein said 2'-deoxynucleotide triphosphate derivative having an azido group has the structure: 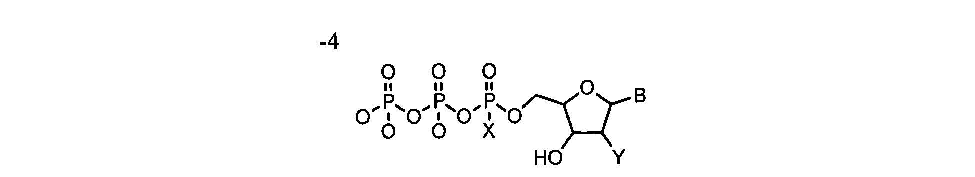 wherein B is selected from the group consisting of A, G, C, T and derivatives thereof, X is O or N₃ and Y is H or N₃ and at least one of X and Y is N₃.
11. A method according to paragraph 1 wherein said derivatized detectable label comprises a phosphone or a click derivative.
12. A method according to paragraph 11 wherein said derivatized detectable label is a phosphone having the structure 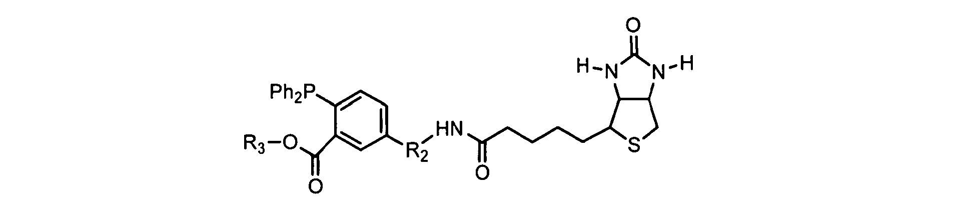 wherein R₂ is a linker, and R₃ is selected from the group consisting of methyl, ethyl, propyl, and iso-propyl.
13. A method according to paragraph 11 wherein said derivatized detectable label is a phosphone having the structure: 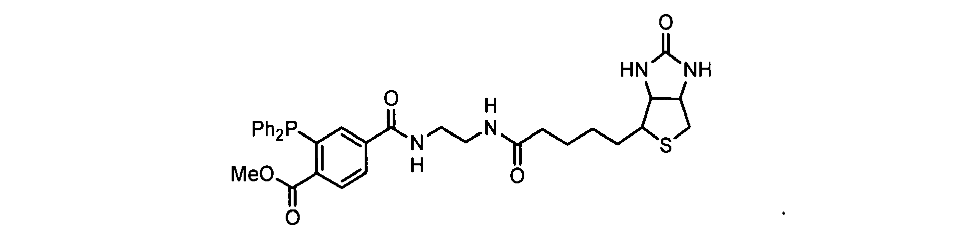
14. A method according to paragraph 11 wherein said derivatized detectable label is a phosphane having the structure 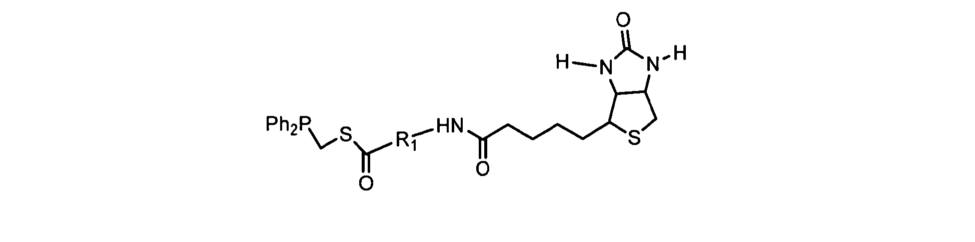 whereR₁ is a linker and R₃ is a linker.
15. A method according to paragraph 14 wherein R₁ is an alkyl linker and R₃ is a linker having a sulfer atom adjacent to the carbonyl group.
16. A method according to paragraph 15 wherein said derivatized detectable label has the structure 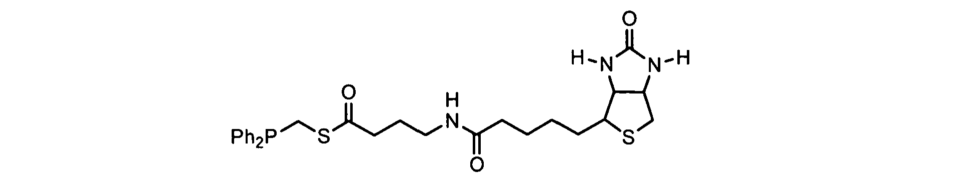
17. A method according to paragraph 1 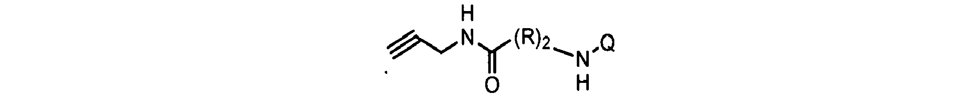 wherein R is a linker and Q is a detectable moiety.
18. A method according to paragraph 17 wherein Q is biotin and R is a water soluble linker having the structure (CH₂CHO)₃CH₂.
19. A method according to paragraph 18 having the structure 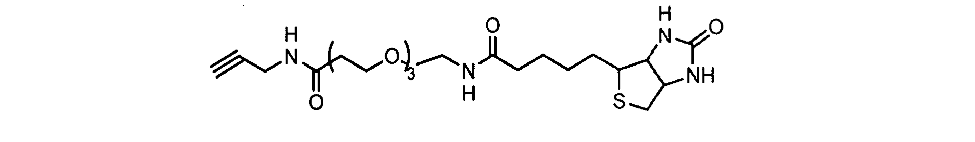
18. A method according to paragraph 17 wherein said click derivatized detectable label has the structure 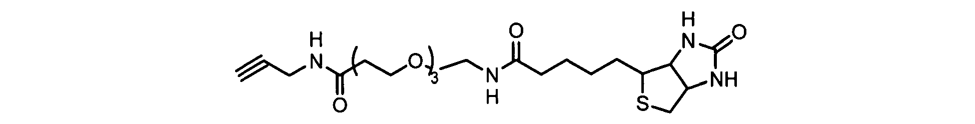
19. A method according to paragraph 1 wherein said 2'-deoxynucleotide triphosphate derivative has the structure: 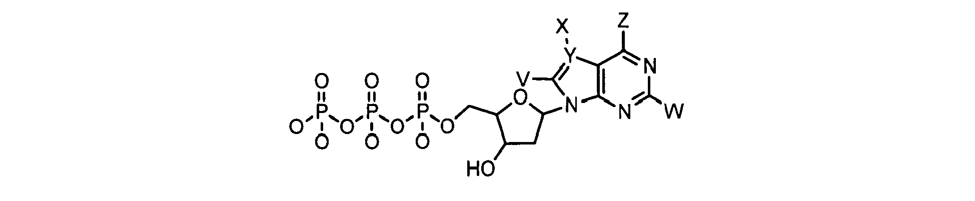 wherein V is H, X is -R-N₃, wherein R is a linker or a bond, Y is N or C, Z is OH, N₃ or NH₂, and W is H, NH₂ or N₃, wherein at least one of X, Z or W is N₃.
20. A method according to paragraph 1 wherein said 2'-deoxynucleotide triphosphate derivative has the structure: 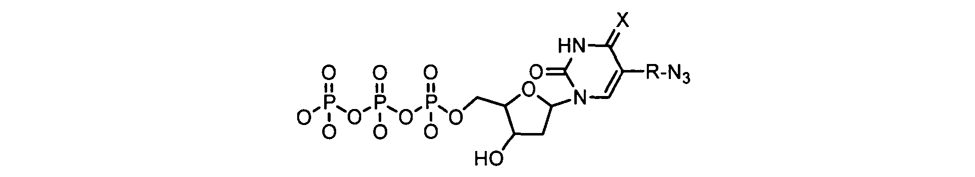 wherein X is NH or O and R is a linker or a bond.
21. A method of according to paragraph 1 wherein said nucleotide derivative has the structure: 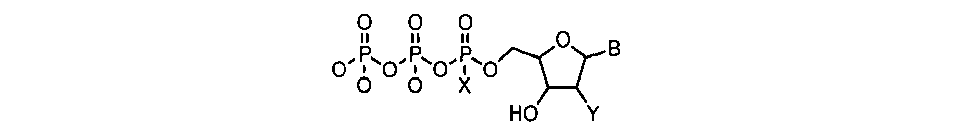 wherein B is selected from the group consisting of A, G, C, T and derivatives thereof, X is O or N₃ and Y is O or N₃ and at least one of X and Y is N₃ and said derivatized detectable label has the structure selected from the group consisting of: 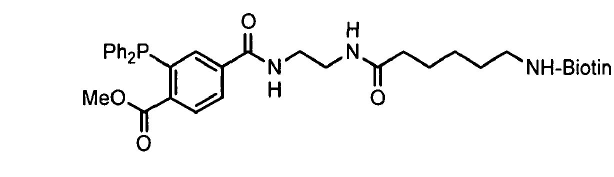 and 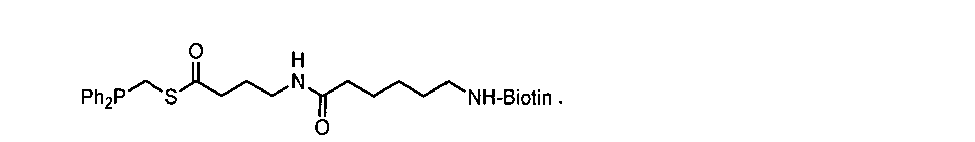
22. A method according to paragraph 19 wherein said nucleotide derivative has the structure: 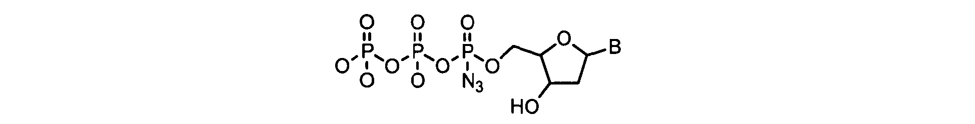 wherein B is selected from the group consisting of A, G, C, T, and said phosphone derivatized detectable label has the structure: 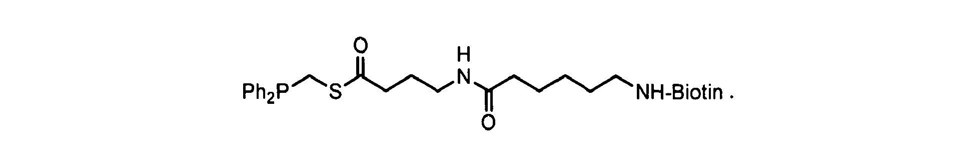
23. A method according to paragraph 1 wherein said labeled DNA has the structure: 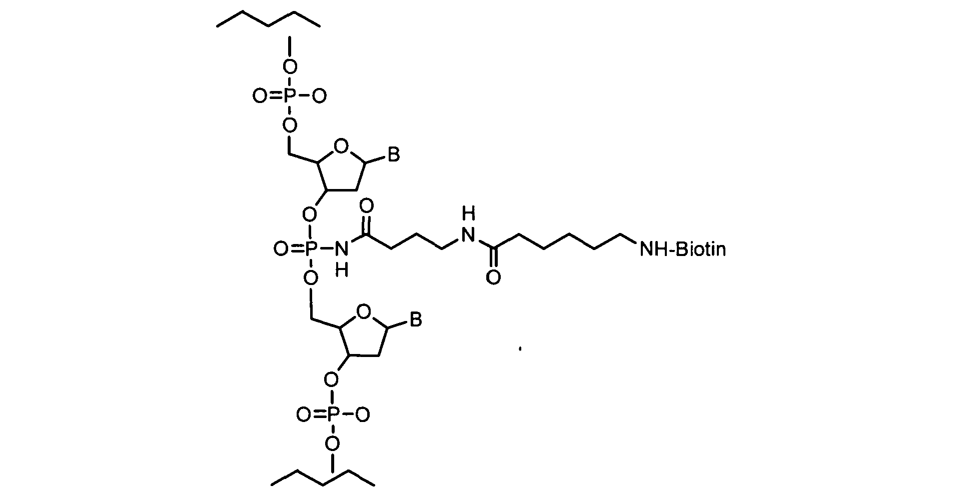 wherein B is a base selected from the group consisting of A, G, T and C
24. A method for detecting the presence or absence of a mRNA in a nucleic acid sample by hybridization to a nucleic acid array, said method comprising the steps of
   providing a nucleic acid sample comprising mRNA;
   hybridizing said mRNA with an oligonucleotide probe comprising a poly dT sequence and a T7 RNA polymerase promoter;
   reverse transcribing said mRNA to provide single stranded DNA;
   converting said single stranded DNA to double stranded DNA wherein said T7 RNA polymerase promoter is oriented to provide cRNA;
   providing a ribonucleotide triphosphate having an orthogonal reactive group which may be incorporated into an RNA strand by a native or mutant T7 RNA polymerase;
   transcribing said double stranded DNA with a natural or mutant T7 RNA polymerase with said ribonucleotide triphosphate having said orthogonal reactive group to provide cRNA having orthogonal reactive groups;
   reacting said orthogonal reactive groups on said cRNA with a derivatized detectable label to provide labeled cRNA; and
   hybridizing said labeled cRNA to said nucleic acid array to detect the presence or absence of said mRNA.
25. A method according to paragraph 24 wherein said T7 RNA polymerase is natural.
26. A method according to paragraph 24 wherein said T7 RNA polymerase is mutant.
27. A method according to paragraph 26 wherein said mutant is Y639F/H784A.
28. A method according to paragraph 24 wherein said orthogonal reactive group comprises an azido group.
29. A method according to paragraph 28 wherein said ribonucleotide triphosphate is selected from the group consisting of 2'-azidoUTP or 2'-azidoCTP.
30. A method for detecting the presence or absence of a polynucleotide of interest on a nucleic acid array, said method comprising the steps of
   providing a nucleic acid sample comprising a polynucleotide;
   providing a nucleotide triphosphate having a reactive orthogonal group;
   enzymatically amplifying said polynucleotide with said nucleotide triphosphate to provide amplified nucleic acids having orthogonal reactive groups;
   reacting said orthogonal groups on said nucleic acids with a detectable label to provide labeled nucleic acids;
   hybridizing said labeled nucleic acids to a nucleic acid array; and
   detecting the presence or absence of said polynucleotide.
31. A method according to paragraph 30 wherein said polynucleotide comprises genomic DNA.
32. A method according to paragraph 30 wherein said polynucleotide comprises mitochondrial DNA.
33. A method according to paragraph 30 wherein said polynucleotide comprises RNA.
34. A method according to paragraph 30 wherein said polynucleotide comprises mRNA.
35. A method according to paragraph 30 wherein said enzyme is selected from the group consisting of an RNA polymerase, a DNA polymerase and a reverse transcriptase.
36. A method according to paragraph 30 wherein said enzyme is selected from the group consisting of a mutant RNA polymerase, a mutant DNA polymerase and a mutant reverse transcriptase.
37. A method according to paragraph 30 wherein said orthogonal group comprises an azido group.
38. A method according to paragraph 37 wherein said nucleotide triphosphate is selected from the group consisting of: 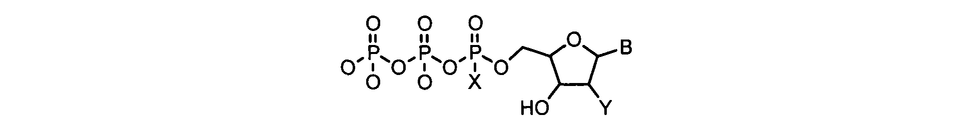 where B is selected from the group of bases consisting of A, G, C, T and derivatives thereof, X is O or N₃ and Y is O or N₃ and at least one of X and Y is N₃; 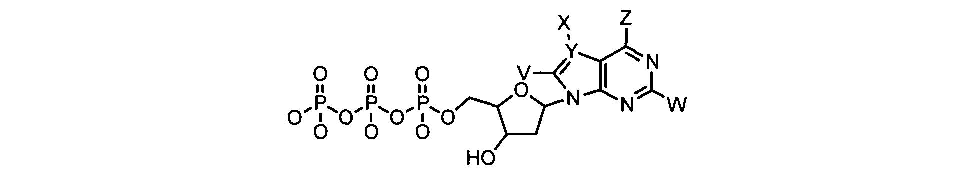 wherein V is H or N₃, X is-R- N₃, wherein R is a linker or a bond, Y is C or N, Z is NH₂, OH or N₃ and W is NH₂, N₃ or H, wherein at least one of V, X, Z or W is N₃; and 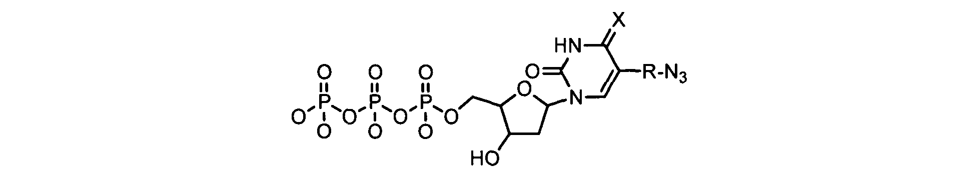 wherein X is NH or O and R is a linker or a bond.
39. A method according to paragraph 30 wherein said detectable label is selected from the group consisting of 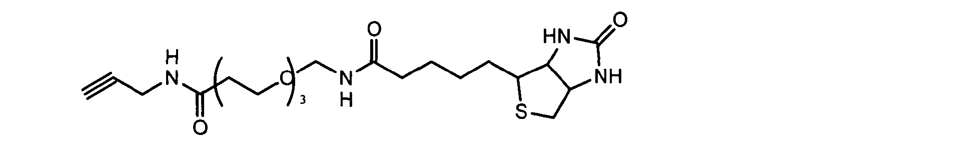 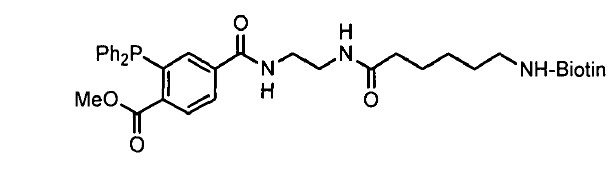 and

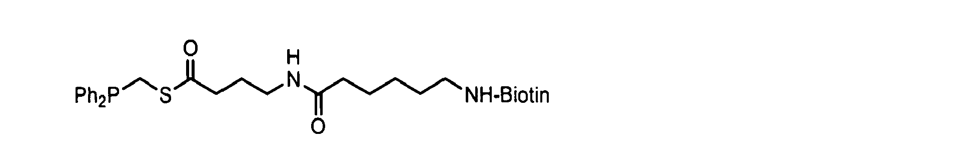

## Claims

1. A method for detecting the presence or absence of a mRNA in a nucleic acid sample by hybridization to a nucleic acid array, said method comprising the steps of
providing a nucleic acid sample comprising mRNA;
hybridizing said mRNA with an oligonucleotide probe homologous to a portion of said mRNA;
providing a 2'-deoxynucleotide triphosphate derivative having an orthogonal group allowing for the specific chemical attachment of a derivatized detectable label;
reverse transcribing said mRNA with a reverse transcriptase to provide reverse transcribed DNA homologous to all or part of said mRNA comprising one or more reactive orthogonal groups;
reacting said orthogonal groups on said DNA with a derivatized detectable label to provide labeled DNA; and
hybridizing said labeled DNA to said nucleic acid array to detect the presence or absence of said mRNA.

2. A method according to claim 1 wherein said reactive orthogonal group is an azido group.

3. A method according to claim 1 or claim 2 wherein said oligonucleotide probe comprises a poly dT sequence, and/or wherein said oligonucleotide probe is from 12-18 nucleotides in length.

4. A method according to claim 1 or claim 2 wherein said step of hybridizing said mRNA with an oligonucleotide probe is carried out by hybridizing said mRNA with a plurality of random primers at least one of which said random primers is homologous to a portion of said mRNA and hybridizes to said mRNA.

5. A method according to claim 4 wherein said random primer is from 6-12 nucleotides in length, optionally wherein said random primer is from 6-9 nucleotides in length, e.g. wherein said random primer is 8 nucleotides.

6. A method according to any one of claims 1 to 5 wherein said 2'-deoxynucleotide triphosphate is an azido-modified nucleotide derivative of the formula
(i) 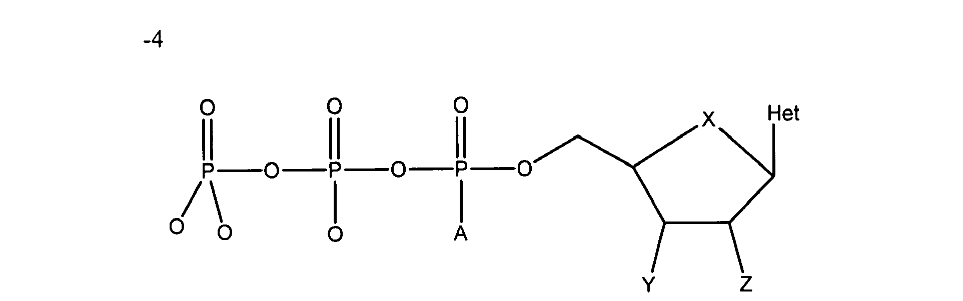 wherein A is O or N₃; X is O, S, NR₁ or CHR₂, wherein R₁ and R₂ are, independently, H, alkyl or aryl; Y is OH; Z is H, N₃, F or OR₁₀, wherein R₁₀ is H, alkyl or aryl, X is O, S, NR₁ or CHR₂, wherein R₁ and R₂ are, independently, H, alkyl or aryl; and Het is a heterocyclic group which is a cyclic moiety containing both carbon and a heteroatom, wherein the heterocyclic group is optionally substituted with N₃ and
wherein at least one of A, Z and Het comprises N₃; or
(ii) 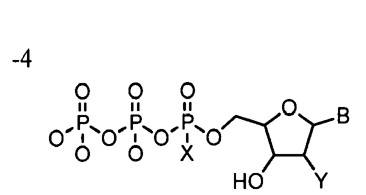 wherein B is selected from the group consisting of A, G, C, T and derivatives thereof,
X is O or N₃ and Y is H or N₃ and at least one of X and Y is N₃.

7. A method according to any one of claims 1 to 6 wherein said derivatized detectable label comprises a phosphone or a click derivative.

8. A method according to claim 7 wherein said derivatized detectable label is a phosphone having the structure
(i) 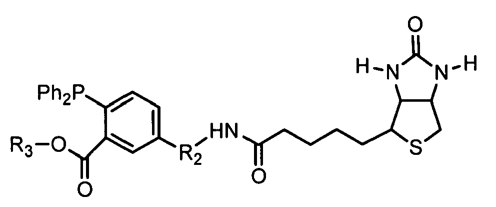 wherein R₂ is a linker, and R₃ is selected from the group consisting of methyl, ethyl, propyl, and iso-propyl;
(ii) 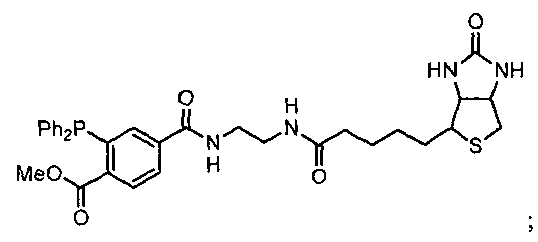 or
(iii) 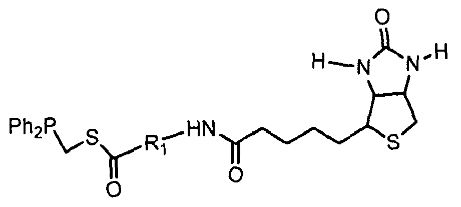 whereR₁ is a linker and.

9. A method according to claim 8(iii) wherein R₁ is an alkyl linker , optionally
wherein said derivatized detectable label has the structure

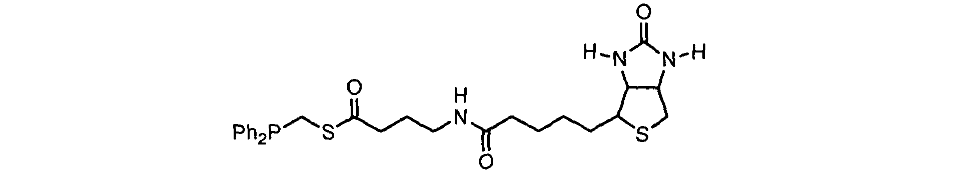

10. A method according to claim 7 wherein said derivatized detectable label is a click derivatized label of the formula 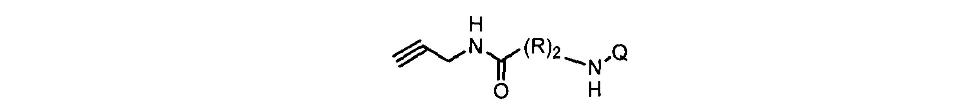 wherein R is a linker and Q is a detectable moiety, optionally wherein Q is biotin and
R is a water soluble linker having the structure (CH₂CHO)₃CH₂.

11. A method according to claim 10 wherein said click derivatized label has the structure
(i) 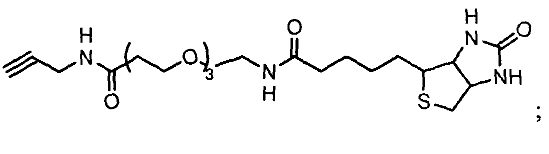 or
(ii)

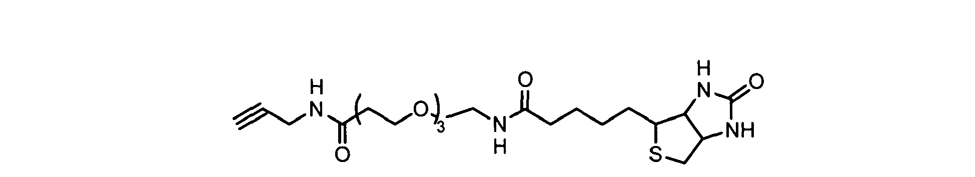

12. A method according to any one of claims 1 to 5 wherein said 2'-deoxynucleotide triphosphate derivative has the structure: 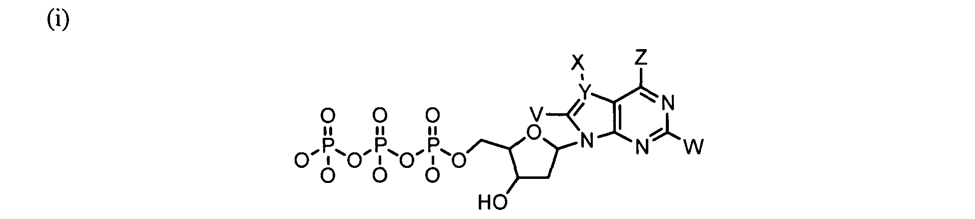 wherein V is H, X is -R-N₃, wherein R is a linker or a bond, Y is N or C, Z is OH, N₃ or NH₂, and W is H, NH₂ or N₃, wherein at least one of X, Z or W is N₃; or 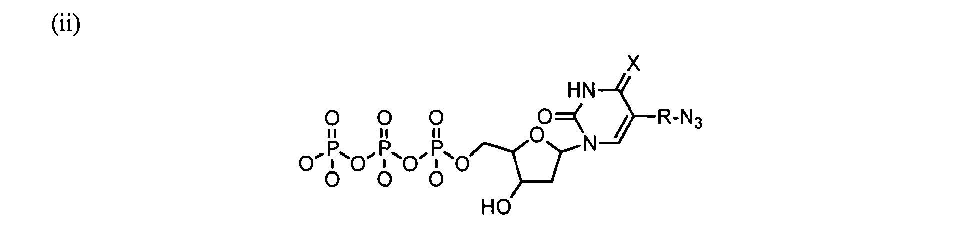 wherein X is NH or O and R is a linker or a bond.

13. A method of according to any one of claims 1 to 5 wherein said nucleotide derivative has the structure: 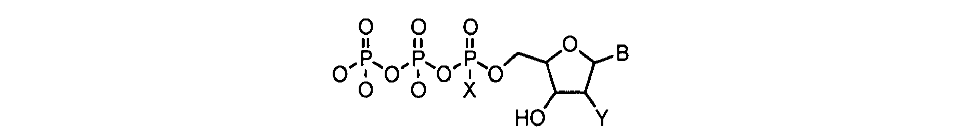 wherein B is selected from the group consisting of A, G, C, T and derivatives thereof,
X is O or N₃ and Y is O or N₃ and at least one of X and Y is N₃ and said derivatized detectable label has the structure selected from the group consisting of: 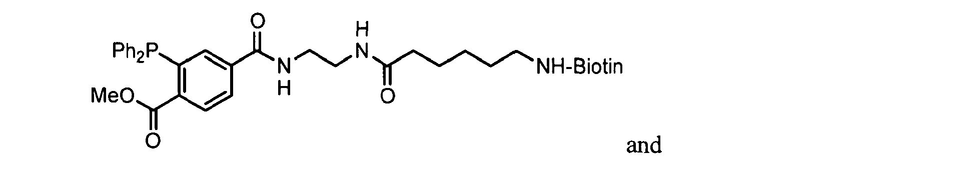 and

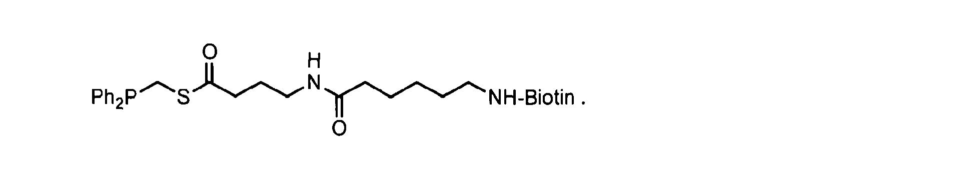

14. A method according to claim any one of claims 1 to 5 wherein said nucleotide derivative has the structure: 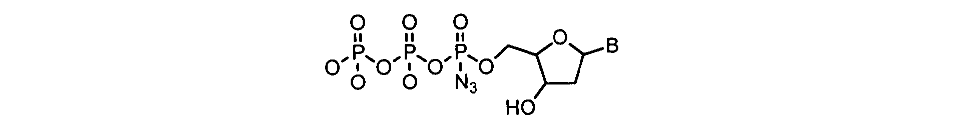 wherein B is selected from the group consisting of A, G, C, T, and said phosphone derivatized detectable label has the structure:

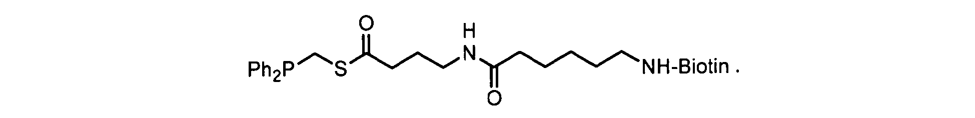

15. A method according to any one of claims 1 to 5 wherein said labeled DNA has the structure: 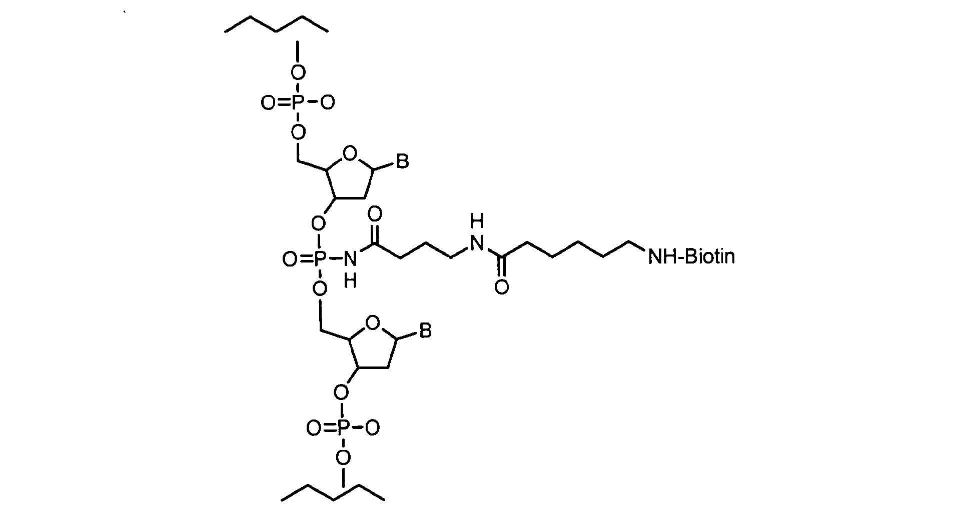 wherein B is a base selected from the group consisting of A, G, T and C

16. A method for detecting the presence or absence of a mRNA in a nucleic acid sample by hybridization to a nucleic acid array, said method comprising the steps of
providing a nucleic acid sample comprising mRNA;
hybridizing said mRNA with an oligonucleotide probe comprising a poly dT sequence and a T7 RNA polymerase promoter;
reverse transcribing said mRNA to provide single stranded DNA;
converting said single stranded DNA to double stranded DNA wherein said T7 RNA polymerase promoter is oriented to provide cRNA;
providing a ribonucleotide triphosphate having an orthogonal reactive group which may be incorporated into an RNA strand by a native or mutant T7 RNA polymerase;
transcribing said double stranded DNA with a natural or mutant T7 RNA polymerase with said ribonucleotide triphosphate having said orthogonal reactive group to provide cRNA having orthogonal reactive groups;
reacting said orthogonal reactive groups on said cRNA with a derivatized detectable label to provide labeled cRNA; and
hybridizing said labeled cRNA to said nucleic acid array to detect the presence or absence of said mRNA.

17. A method according to claim 16 wherein said T7 RNA polymerase is the mutant Y639F/H784A.

18. A method according to claim 16 or claim 17 wherein said orthogonal reactive group comprises an azido group, optionally wherein said ribonucleotide triphosphate is selected from the group consisting of 2'-azidoUTP or 2'-azidoCTP.

19. A method for detecting the presence or absence of a polynucleotide of interest on a nucleic acid array, said method comprising the steps of
providing a nucleic acid sample comprising a polynucleotide;
providing a nucleotide triphosphate having a reactive orthogonal group;
enzymatically amplifying said polynucleotide with said nucleotide triphosphate to provide amplified nucleic acids having orthogonal reactive groups;
reacting said orthogonal groups on said nucleic acids with a detectable label to provide labeled nucleic acids;
hybridizing said labeled nucleic acids to a nucleic acid array; and
detecting the presence or absence of said polynucleotide.

20. A method according to claim 19 wherein said polynucleotide is selected from the group consisting of genomic DNA, mitochondrial DNA, RNA and mRNA.

21. A method according to claim 19 wherein said enzyme is selected from the group consisting of an RNA polymerase, a DNA polymerase, a reverse transcriptase, mutant RNA polymerase, a mutant DNA polymerase and a mutant reverse transcriptase.

22. A method according to any one of claims 19 to 21 wherein said orthogonal group comprises an azido group.

23. A method according to claim 22 wherein said nucleotide triphosphate is selected from the group consisting of:
(i) 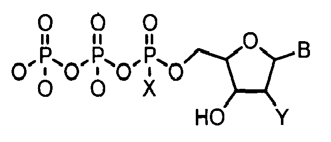 where B is selected from the group of bases consisting of A, G, C, T and derivatives thereof, X is O or N₃ and Y is O or N₃ and at least one of X and Y is N₃;
(ii) 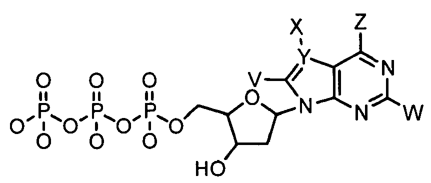 wherein V is H or N₃, X is-R- N₃, wherein R is a linker or a bond, Y is C or N, Z is NH₂, OH or N₃ and W is NH₂, N₃ or H, wherein at least one of V, X, Z or W is N₃; and
(iii) 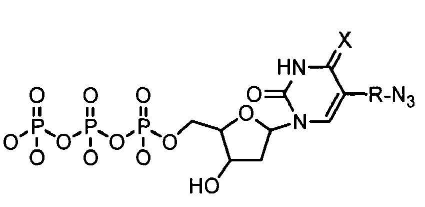 wherein X is NH or O and R is a linker or a bond.

24. A method according to any one of claims 19 to 23 wherein said detectable label is selected from the group consisting of 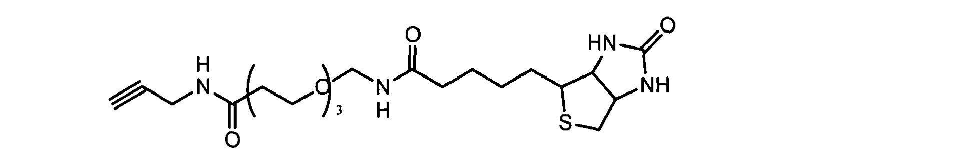 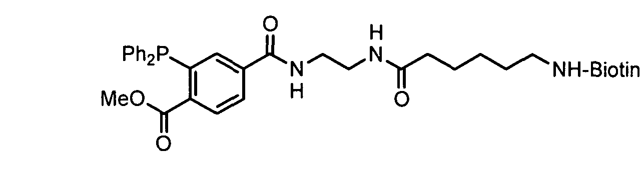 and

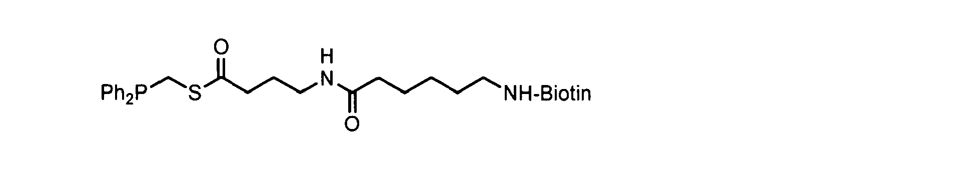

25. Use of a nucleotide triphosphate derivative having an orthogonal group, e.g. an azido group, allowing for the specific chemical attachment of a derivative detectable label, in detection of a polynucleotide, optionally in detection of a polynucleotide on a nucleic acid array.

26. Use according to claim 25 wherein said nucleotide triphosphate derivative is as defined in any one of claims 6, 12, 13, 14 and 23.

27. Use of a derivatized detectable label and a nucleotide triphosphate derivative having an orthogonal group, e.g. an azido group, allowing for the specific chemical attachment of said derivatised detectable label, for detection of a polynucleotide, optionally for detection of a polynucleotide or a nucleic acid array.

28. Use according to claim 27, wherein said nucleotide triphosphate derivative is as defined in any one of claims 12, 13, 14 and 23 and wherein said derivatized detectable label is as defined in any one of claims 7 to 11, 13, 14 and 24.
